# EUROPEAN PATENT APPLICATION

(11) **EP 3 738 603 A1**
(43) Date of publication of application: **18.11.2020**
(21) Application number: 19738828.3
(22) Date of filing: 11.01.2019
(51) Int. Cl.: A61K 38/12, A61P 1/16, A61P 31/12, A61P 31/14, A61P 31/20, A61P 43/00, C07K 5/04, C07K 7/06, C07K 7/08

(54) **NTCP INHIBITOR**

(30) Priority: 11.01.2018 JP 2018002814
(71) Applicant: The University of Tokyo, Bunkyo-ku, Tokyo 113-8654 (JP); Japan as Represented by The Director-General of National Institute of Infectious Diseases, Tokyo 162-8640 (JP)
(72) Inventor: SUGA, Hiroaki, Tokyo 113-8654 (JP); PASSIOURA, Toby, Tokyo 113-8654 (JP); WAKITA, Takaji, Tokyo 162-8640 (JP); WATASHI, Koichi, Tokyo 162-8640 (JP)
(74) Representative: Mewburn Ellis LLP
(86) International application number: PCT/JP2019/000699
(87) International publication number: WO 2019/139126

(57) **Abstract**

The present invention provides a sodium taurocholate cotransporting polypeptide (NTCP) inhibitor comprising a cyclic peptide having a Xw1-W-Xw2-W structure, wherein Xw1 and Xw2 are each independently T, S, I, L, or V, or a N-alkylamino acid thereof, or having a P-Xp1-Xp2-H structure, wherein Xp1 is any amino acid, and Xp2 is I, L, or V, or a N-alkylamino acid thereof, or a pharmaceutically acceptable salt thereof.

## Description

### Technical Field

The present invention relates to a NTCP inhibitor.

### Background Art

As a few currently available methods for treating hepatitis B virus (HBV) are effective, the development of novel treatment methods is in demands. In this context, it has been known in recent years that hepatitis B virus (HBV) or hepatitis D virus (HDV) infects humans by entry into cells via sodium taurocholate cotransporting polypeptide (NTCP) and develops hepatitis B. In response to this, for example, use of cyclosporin A (CsA) or myrcludex B has been studied as an attempt to treat hepatitis B by inhibiting NTCP (e.g., Non Patent Literature 1).

### Citation List

### Non Patent Literature

[Non Patent Literature 1] Nkongolo, S. et al. Cyclosporin A inhibits hepatitis B and hepatitis D virus entry by cyclophilin-independent interference with the NTCP receptor. Journal of hepatology 60, 723-731 (2014).
[Non Patent Literature 2] Ni Y et al. Hepatitis B and D viruses exploit sodium taurocholate co-transporting polypeptide for species-specific entry into hepatocytes. Gastroenterology 146: 1070-1083 (2014).
[Non Patent Literature 3] Yan H et al. Viral entry of hepatitis B and D viruses and bile salts transportation share common molecular determinants on sodium taurocholate cotransporting polypeptide. J Virol 88: 3273-3284 (2014).
[Non Patent Literature 4] Watashi K et al. Interleukin-1 and tumor necrosis factor-alpha trigger restriction of hepatitis B virus infection via a cytidine deaminase activation-induced cytidine deaminase (AID). Hepatology 59: 1726-1737 (2014).
[Non Patent Literature 5] Blank A et al. The NTCP-inhibitor Myrcludex B: Effects on Bile Acid Disposition and Tenofovir Pharmacokinetics. Clin Pharmacol Ther 00: 1-8 (2017)

### Summary of Invention

### Technical Problem

However, these conventional inhibitors not only inhibit HBV or HDV entry into cells via NTCP but inhibit even the original functions of NTCP, such as transportation of bile acid (e.g., Non Patent Literatures 2 to 5).

Accordingly, an object of the present invention is to provide a novel NTCP inhibitor based on a cyclic peptide.

### Solution to Problem

The present inventors have conducted diligent studies to attain the object and consequently completed the present invention by finding that a cyclic peptide having a specific structure inhibits HBV or HDV entry into cells via NTCP.

Specifically, the present invention is as given below.
[1] A sodium taurocholate cotransporting polypeptide (NTCP) inhibitor comprising a cyclic peptide having a Xw1-W-Xw2-W structure, wherein Xw1 and Xw2 are each independently T, S, I, L, or V, or a N-alkylamino acid thereof, or having a P-Xp1-Xp2-H structure, wherein Xp1 is any amino acid, and Xp2 is I, L, or V, or a N-alkylamino acid thereof, or a pharmaceutically acceptable salt thereof.
[2] The NTCP inhibitor according to [1], wherein the cyclic peptide has a Xw1-W-Xw2-W-Xw3-W structure, wherein Xw1 and Xw2 are as defined above, and Xw3 is T, S, I, L, or V, or a N-alkylamino acid thereof.
[3] The NTCP inhibitor according to [1] or [2], wherein Xw1 is T or L, and Xw2 is T or I.
[4] The NTCP inhibitor according to [1], wherein the cyclic peptide has a P-Xp1-Xp2-H-Xp3-F structure, wherein Xp1 and Xp2 are as defined above, and Xp3 is I, L, or V, or a N-alkylamino acid thereof.
[5] The NTCP inhibitor according to [4], wherein Xp2 and Xp3 are each independently I or L.
[6] The NTCP inhibitor according to any of [1] to [5], wherein the cyclic peptide has a N-CO-CH₂-S structure.
[7] The NTCP inhibitor according to [6], wherein the N is derived from an amino group of tryptophan.
[8] The NTCP inhibitor according to [6] or [7], wherein the S is derived from a thiol group of cysteine.
[9] The NTCP inhibitor according to any of [1] to [8], wherein the cyclic peptide has a cyclic structure having from 10 to 20 amino acids.
[10] The NTCP inhibitor according to any of [1] to [9], wherein the NTCP is human NTCP.
[11] The NTCP inhibitor according to any of [1] to [10], wherein the NTCP inhibitor is an inhibitor of entry of hepatitis B virus (HBV) or hepatitis D virus (HDV) into cells.
[12] A pharmaceutical composition comprising a NTCP inhibitor according to any one of [1] to [11].
[13] The pharmaceutical composition according to [12] for use in treatment or prevention of a disease related to hepatitis B virus or hepatitis D virus.

### Advantageous Effects of Invention

The present invention can provide a novel NTCP inhibitor based on a cyclic peptide.

### Brief Description of Drawings

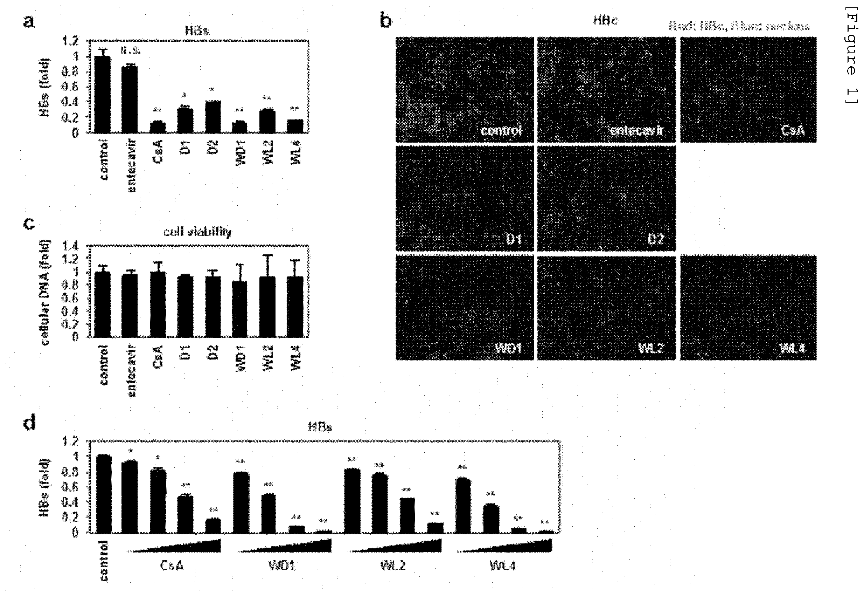

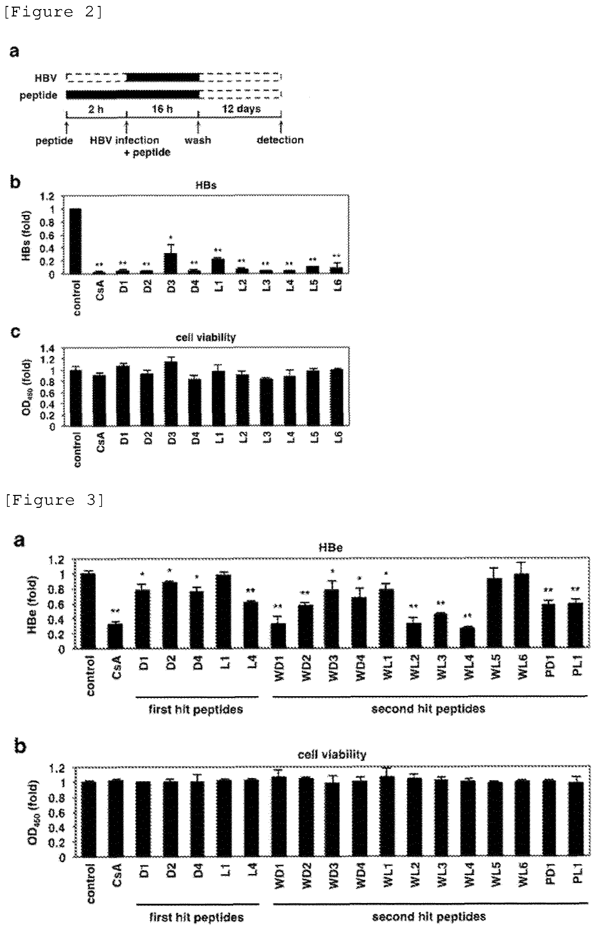

[Figure 1] Figure 1 is a diagram showing the anti-HBV activity of a selected cyclic peptide. In Figures 1(a) to 1(c), HepG2-hNTCP-C4 cells were treated with 100 nM entecavir, 30 µM cyclosporin A (CsA), a peptide (D1 and D2) or a peptide identified from a focused library (WD1, WL2 and WL4) (shown in Figure 2(a)). HBs antigen secreted into a culture supernatant (a) and HBc antigen in the cells (b) were detected by ELISA and an immunofluorescence method, respectively. A cell survival rate was measured by quantifying total cellular DNA (c). The staining images of (b) each show the HBc antigen and the nucleus. In Figure 1(d), HepG2-hNTCP-C4 cells were treated with varying concentrations (0.3, 1, 3 and 10 µM) of CsA or a peptide (WD1, WL2 or WL4) as shown in Figure 2(a). HBs antigen secreted into a culture supernatant was detected by ELISA. Data is indicated by mean ± SD. Statistical significance was determined by use of Student's t test as to all numbers (*P < 0.05, **P < 0.01, N.S.: not significant).
[Figure 2] Figure 2 is a diagram showing the anti-HBV effect of a peptide from initial screening. Figure 2(a) is a schematic diagram of a HepG2-hNTCP-C4 infection experiment. HepG2-hNTCP-C4 cells were pretreated with a peptide for 2 hours and subsequently inoculated with HBV for 16 hours in the presence of the peptide. Free HBV and peptide were washed off. Then, the cells were further cultured for 12 days in the absence of the peptide, and HBV infection was evaluated on the basis of measurement of HBs antigen in the culture supernatant. The filled boxes and the broken-line boxes depict periods for which the treatment was performed and periods for which the treatment was not performed, respectively. In
Figure 2(b), primary human hepatocytes were treated with 100 µM CsA or a peptide identified from the first screening, as shown in Figure 2(a). HBV infection was evaluated by monitoring HBs antigen secreted into a culture supernatant on 12 days after inoculation by ELISA. In Figure 2(c), a cell survival rate was measured by MTT assay.
[Figure 3] Figure 3 is a diagram showing the anti-HBV effect of a peptide by focused library screening. In Figures 3(a) and 3(b), primary human hepatocytes or HepG2-hNTCP-C4 cells were treated with 10 µM CsA or a shown cyclic peptide according to the protocol shown in Figure 2(a). HBV infection was evaluated by measurement of HBe antigen (a), and a cell survival rate (b) was evaluated by MTT assay as shown in Figure 2.

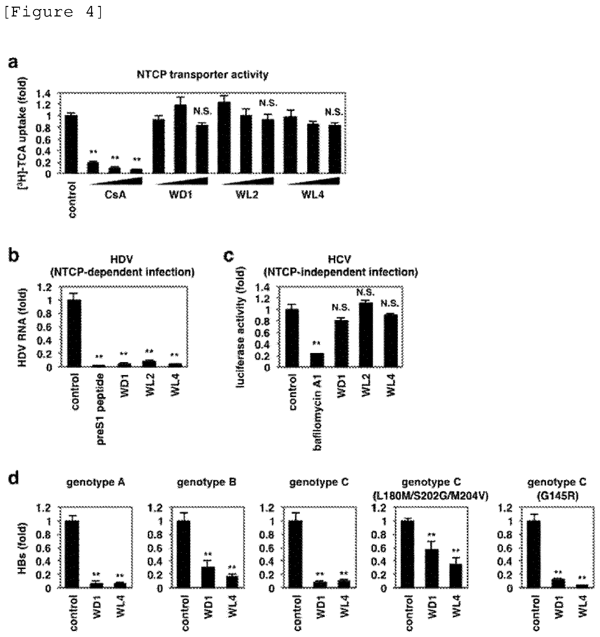

[Figure 4] Figure 4 is a diagram showing the inhibition of NTCP transporter activity by a selected cyclic peptide and the anti-HBV activity of the peptide against a wide range of genotypes. In Figure 4(a), bile acid uptake was quantified as [³H] uptake by incubating HepG2-hNTCP-C4 cells with [³H]-taurocholic acid (TCA) as a substrate in a sodium-containing buffer solution in the presence of varying concentrations (3, 10, and 30 µM) of CsA, WD1, WL2 or WL4. In Figure 4(b), HepG2-hNTCP-C4 cells were treated with HDV together with 100 nM preS1 peptide (positive control) or 30 µM WD1, WL2 or WL4 for 16 hours. Free HDV and peptide were washed. Then, the cells were further cultured for 6 days in the absence of the peptide, and the amount of intracellular HDV RNA was quantified by real-time RT-PCR analysis. In Figure 4(c), Huh-7.5.1 cells were treated with 20 nM bafilomycin A1 (positive control) or 30 µM WD1, WL2 or WL4 and inoculated with HCV pseudoparticles for 4 hours. 72 hours after injection, intracellular luciferase activity was measured, and HCV infection was evaluated. In Figure 4(d), primary human hepatocytes were treated with 100 nM preS1 peptide (positive control) or 30 µM WD1 or WL4, and different HBV isolates (genotypes A, B, and C and genotype C having L180M/S202G/M204V mutations or a G145R variant) were isolated according to the protocol shown in Figure 2(a). HBV infection was evaluated by detecting HBs antigen secreted into a culture supernatant.

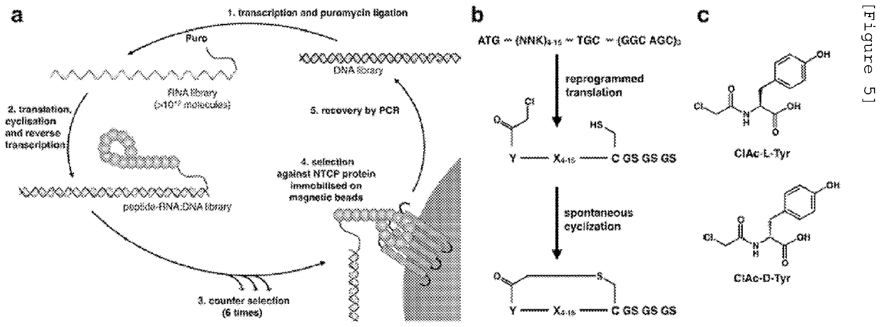

[Figure 5] Figure 5 is a diagram showing a strategy of cyclic peptide screening. Figure 5(a) is a diagram showing the outline of a RaPID screening approach: (1) a DNA library assembled from synthetic oligonucleotides was transcribed into RNA, which was then 3'-terminally ligated to puromycin. (2) Translation of this library in genetically reprogrammed translation reaction led to the formation of a cyclic peptide library in which each peptide was covalently bound via the puromycin moiety to its cognate mRNA, which was then reverse transcribed to generate combined peptide-RNA:DNA molecules. (3) Counter selections were used to remove peptides that bound non-specifically to bead surface, followed by isolation of NTCP-binding peptides through panning of the library against NTCP immobilized on magnetic beads. (4) An enriched DNA library was recovered by PCR, and the process was repeated until increased rates of target binding were observed. Deconvolution of the library was achieved by sequencing of the final (and intermediate) enriched DNA libraries. In Figure 5(b), a cyclic thioester strategy was used: in a semi-randomized RNA library composed of a start codon and a variable (4 to 15 cod-ons) NNK codon region (N = any amino acid, and K = U or G), a Cys codon and a Gly-Ser linker sequence were reprogrammed such that the initiating amino acid was N-chloroacetyl-tyrosine (ClAc-Tyr), which spontaneously reacts with the downstream Cys after translation to yield a thioether cyclized peptide. In Figure 5(c), libraries initiated with either L- or D-ClAc-Tyr were used.

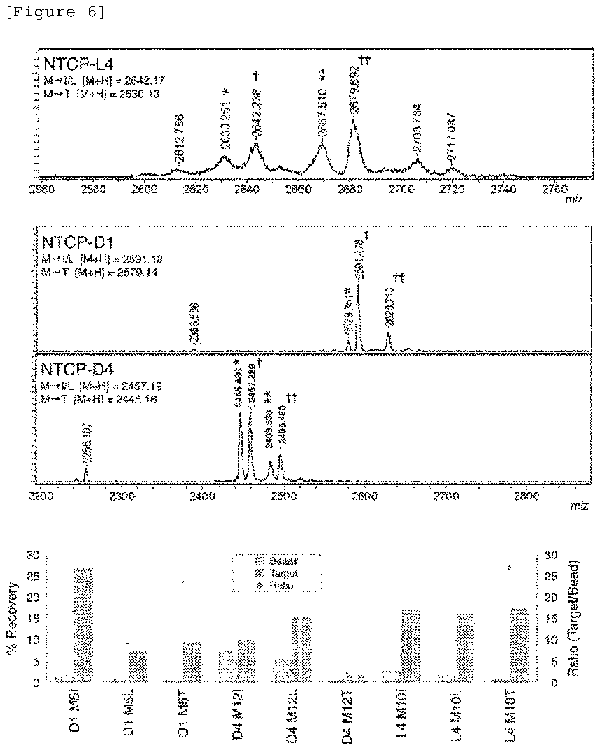

[Figure 6] Figure 6 is a diagram showing evaluation of Met codon misreading in an identified peptide. Some of peptides identified from first screening as to NTCP affinity contained a Met codon, though Met was absent in translation reaction. In order to evaluate what amino acid was mistakenly read as the Met codon, each cDNA was translated through Met-deficient translation reaction, and the mass thereof was determined by MALDI-TOF MS. Misreads corresponding to Thr (*) or Ile/Leu (†) and K adducts corresponding thereto (** and ††) were observed. In order to evaluate the influence of each misread on NTCP binding, individual clones were synthesized by primer assembly, and their affinity for magnetic beads or NTCP immobilized on each bead was evaluated as to affinity selection itself (i.e., related mRNA puromycin, *in vitro* translation, affinity selection and quantification by real-time RT-PCR). D1 (M5I), D4 (M12L) and L4 (M10T) were all selected for subsequent studies.

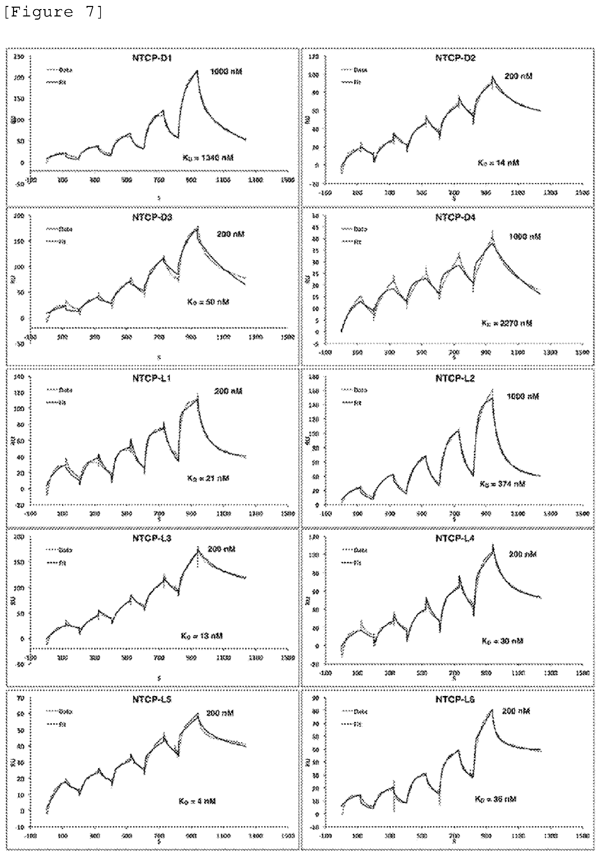

[Figure 7] Figure 7 is a diagram showing the NTCP association constant of a peptide identified by first RaPID screening. A single-cycle reaction kinetics experiment was conducted using NTCP immobilized on surface (NTA-Ni²⁺) involving varying concentrations of a cyclic peptide analyte. In each case, as shown in the drawing, the peptide was tested with 4-fold serial dilutions starting from the highest concentration of 200 or 1000 nM. A dissociation constant (K_{D}) was determined using a 1:1 binding model.

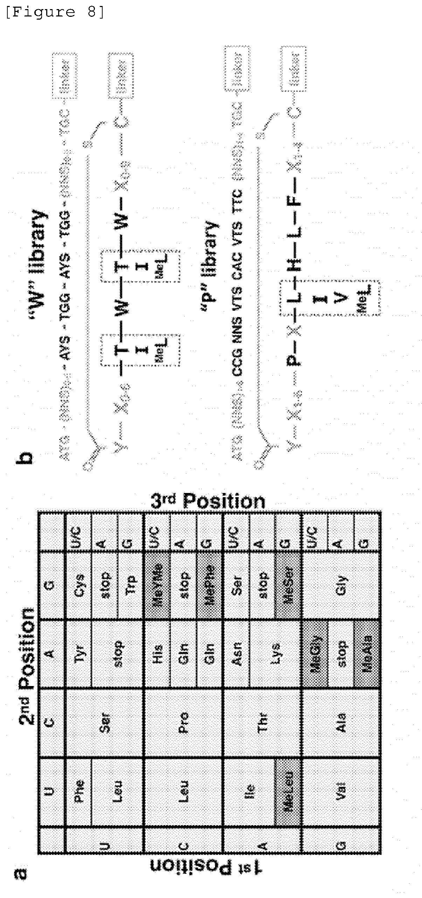

[Figure 8] Figure 8 is a diagram showing one example of design of an α-N-methylated, focused peptide library. Figure 8(a) is a codon table for translation of focused libraries containing MeLeu = N-methyl-leucine, MeGly = N-methyl-glycine, MeAla = N-methylalanine, and MeYMe = N-methyl-4-phenylalanine. MePhe = N-methyl-phenylalanine, MeSer = N-methyl-serine). Figure 8(b) shows design of focused libraries based on consensus of initial screen hits: two libraries, "W" library and "P" library, were based on (T/L)W(T/I)W and PX(I/L)H(I/L)F motifs, respectively. Each library contained an initiating ClAc-Tyr residue, followed by a semi-randomized region, a Cys residue and a Thr-Gln linker. The "W" library was based on an AYS-TGG-AYS-TGG DNA motif (Y = C or T, S = C or G) flanked by a variable number of NNS codons (encoding any amino acid in the panel a; designated as "X" such that Thr, Ile, or MeLeu could be translated in the conserved motif). The P library was based on a CCG-NNS-VTS-CAC-VTS-TTC DNA motif (V = C, A, or G, S = C or G) flanked by a variable number of NNS codons (encoding any amino acid; designated as "X" in the panel a such that Leu, Ile, Val, or MeLeu could be translated in the conserved motif).

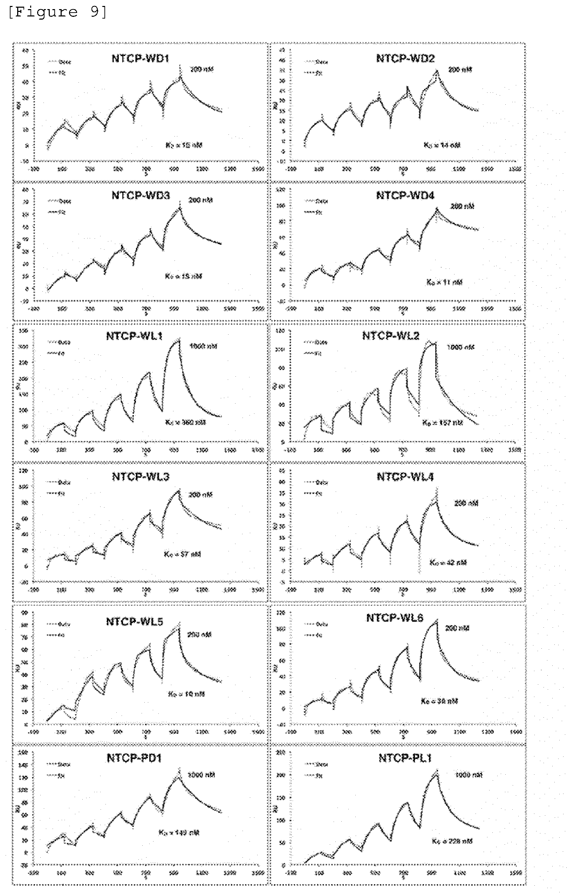

[Figure 9] Figure 9 is a diagram showing the NTCP association constant of a peptide identified by focused library RaPID screening. A single-cycle association constant experiment was conducted using NTCP immobilized on surface (NTA-Ni²⁺) involving varying concentrations of a cyclic peptide analyte. In each case, as shown in the drawing, the peptide was tested with 4-fold serial dilutions starting from the highest concentration of 200 or 1000 nM. A dissociation constant (K_{D}) was determined using a 1:1 binding model.

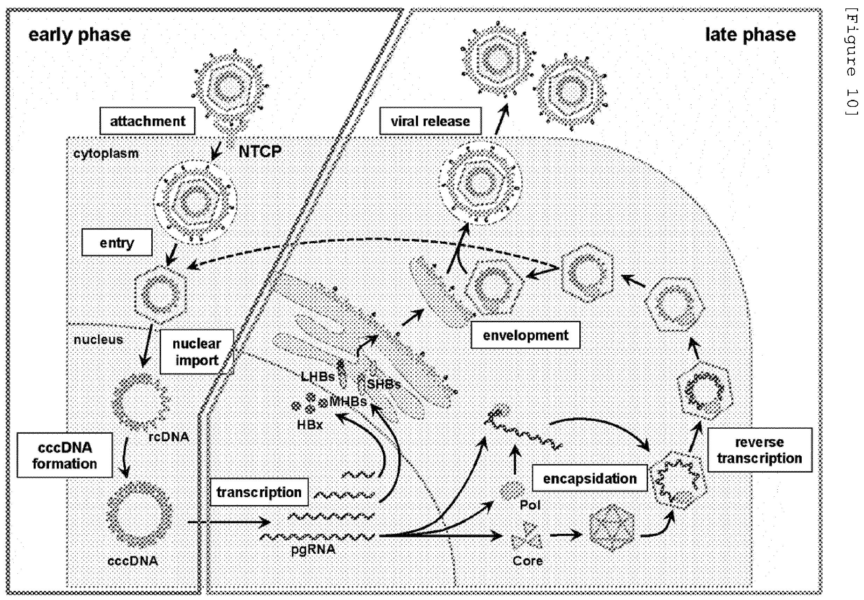

[Figure 10] Figure 10 is a diagram showing the outline of a HBV life cycle. The HBV life cycle is divided into an early phase of infection involving attachment, entry, nuclear import, and cccDNA formation, and a late phase involving transcription, encapsidation, reverse transcription, envelopment and viral release.

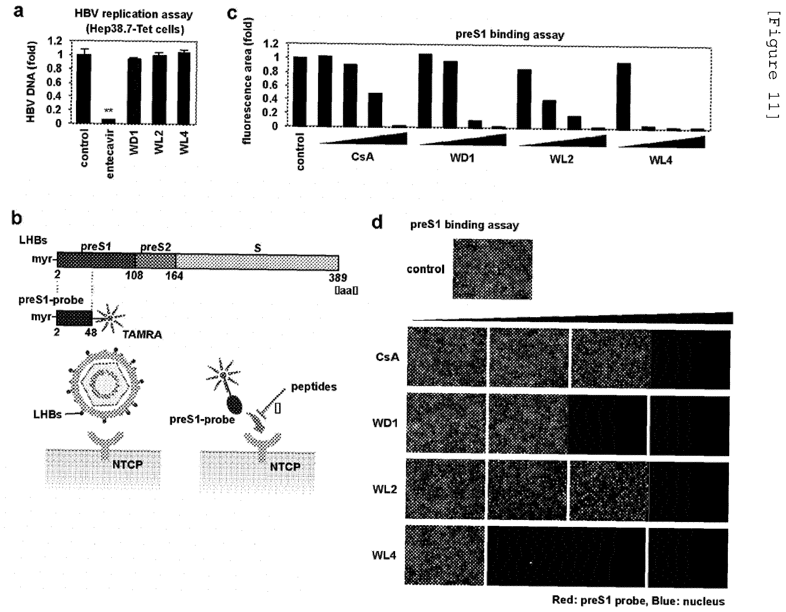

[Figure 11] Figure 11 is a diagram showing the inhibition of HBV entry by a selected cyclic peptide. In Figure 11(a), HBV replication was evaluated by real-time PCR analysis of HBV DNA in Hep38.7-Tet cells treated with 100 nM entecavir or 30 µM each peptide for 6 days after tetracycline withdrawal. Figure 11(b) is a schematic diagram of preS1 binding assay using fluorescent preS1 probes (amino acids 2 to 48) to evaluate HBV attachment to host cells. In Figures 11(c) and 11(d), PreS1 attachment was evaluated in HepG2-hNTCP-C4 cells at 37°C for 30 minutes with varying concentrations (1, 3, 10 and 30 µM) of CsA, WD1, WL2 or WL4. Red and blue signals depict the preS1 probes and the nucleus, respectively. PreS1 fluorescence intensity (d) was quantified using a dynamic cell count (Keyence Corp.) and is shown in Figure 11(c).

### Description of Embodiments

The present invention will be specifically described with reference to the mode for carrying out the invention. However, the present invention is not limited by the following mode for carrying out the invention and can be carried out through various changes or modifications.

The contents disclosed in the references described in the present invention are incorporated herein by reference.

In the present specification, the "cyclic peptide" means a peptide having at least a cyclic structure formed from 4 or more amino acids in the molecule. The cyclic peptide may have a chain-like structure where amino acids are connected through peptide bonds, in addition to the cyclic structure, as a molecular structure. Also, the cyclic peptide may have a structure other than the peptide structure.

In the present specification, the "cyclic structure" means a closed cyclic structure formed in the molecule through direct binding or linker-mediated binding, etc. of 2 amino acids distant by 2 or more amino acid residues in a linear peptide.

In the present specification, the phrase "distant by 2 or more amino acid residues" means that at least 2 residues of amino acids are present between 2 amino acids.

The closed cyclic structure in the cyclic structure is not particularly limited and is formed through covalent binding of 2 amino acids.

Examples of the covalent binding between 2 amino acids include disulfide bonds, peptide bonds, alkyl bonds, alkenyl bonds, ester bonds, thioester bonds, ether bonds, thioether bonds, phosphonate ether bonds, azo bonds, C-S-C bonds, C-N-C bonds, C=N-C bonds, amide bonds, lactam cross-links, carbamoyl bonds, urea bonds, thiourea bonds, amine bonds, and thioamide bonds.

When 2 amino acids are bound at their backbones, a closed cyclic structure is formed through a peptide bond. The covalent binding between 2 amino acids may be formed through a bond between the side chains of the 2 amino acids, between the side chain and the backbone of the 2 amino acids, etc.

The cyclic structure may be formed not only through the binding of the N-terminal and C-terminal amino acids of the linear peptide but through the binding of a terminal amino acid to a non-terminal amino acid or the binding of non-terminal amino acids. When one of the amino acids to be bound for the formation of the cyclic structure is a terminal amino acid and the other amino acid is a non-terminal amino acid, the cyclic peptide has a structure of the cyclic structure tailed with a linear peptide.

The amino acids constituting the cyclic structure include natural amino acids as well as artificial amino acid variants and derivatives. Examples thereof include natural proteinogenic L-amino acids, non-natural amino acids, and chemically synthesized compounds having characteristics known in the art which are features of amino acids.

The proteinogenic amino acids are Arg, His, Lys, Asp, Glu, Ser, Thr, Asn, Gln, Cys, Gly, Pro, Ala, Ile, Leu, Met, Phe, Trp, Tyr, and Val, which are indicated by three-letter code well known in the art.

The non-proteinogenic amino acids mean natural or non-natural amino acids other than the proteinogenic amino acids.

Examples of the non-natural amino acids include amino acids differing in backbone structure from the natural ones, such as α,α-disubstituted amino acids (α-methylalanine, etc.), N-alkyl-α-amino acids, D-amino acids, β-amino acids, and α-hydroxylic acid, amino acids differing in side chain structure from the natural ones (norleucine, homohistidine, etc.), amino acids having extra methylene in a side chain ("homo" amino acids, homophenylalanine, homohistidine, etc.), and amino acids in which a carboxylic acid functional group in a side chain is substituted by a sulfonic acid group (cysteic acid, etc.). Specific examples of the non-natural amino acids include amino acids described in International Publication No. WO 2015/030014.

The number of amino acids constituting the cyclic structure is not particularly limited as long as the number of amino acids is 4 or more. The number of amino acids may be, for example, 5 or more, 8 or more, or 10 or more and may be 30 or less, 25 or less, 20 or less, or 15 or less.

The number of amino acids constituting the cyclic structure is preferably 4 or more and 30 or less. The number of amino acids constituting the cyclic structure may be 5 or more, 8 or more, or 10 or more and may be 30 or less, 25 or less, 20 or less, or 15 or less, within the range of 4 or more and 30 or less.

The number of amino acids constituting the cyclic structure may be 8 or more and 20 or less, may be 10 or more and 20 or less, and may be 10 or more and 15 or less.

In the present invention, the cyclic peptide may be subjected to a modification such as phosphorylation, methylation, acetylation, adenylylation, ADP ribosylation, or glycosylation, or may be fused with an additional peptide or protein. Also, the cyclic peptide may be biotinylated via an appropriate linker.

In the present invention, the cyclic peptide may be a dimer having 2 cyclic structures in the molecule where 2 cyclic peptides each having 1 cyclic structure are bound via a linker structure, or may have an intramolecular lactam bridge structure where a lactam structure is formed in the molecule.

The linker structure that connects 2 cyclic peptides is not particularly limited, and a structure well known as a linker that connects peptides in the peptide synthesis field can be adopted.

The intramolecular lactam bridge structure can be formed through the binding of side chains of amino acids constituting the cyclic peptide. The intramolecular lactam structure is formed, for example, by formation of a peptide bond through the binding of a side chain amino group of Lys to a side chain carboxyl group of Asp or Glu so that the cyclic peptide has another cyclic structure as a bridge structure in the molecule. For example, DAP, DAB, and Orn may be bound, instead of Lys, to Asp or Glu.

The present invention relates to a novel NTCP inhibitor based on a cyclic peptide. Particularly, the present invention relates to a NTCP inhibitor having a weaker inhibitory effect on the original functions of NTCP, a NTCP inhibitor more strongly inhibiting the interaction between HBV or HDV and NTCP, or a NTCP inhibitor serving as both of them.

In the present invention, a cyclic peptide having a Xw1-W-Xw2-W structure or having a P-Xp1-Xp2-H structure is considered to allosterically inhibit at least a portion of the functions of NTCP by binding to NTCP via the Xw1-W-Xw2-W structure or the P-Xp1-Xp2-H structure. Among others, the NTCP inhibitor according to the present invention suitably functions as an entry inhibitor for inhibiting HBV or HDV entry into cells.

A HBV life cycle is generally divided into an early stage of infection and a late stage of infection for cells. More specifically, at the early stage of infection, HBV binds to NTCP on hepatocytes and enters cells via the NTCP, followed by transportation to the nuclei to form cccDNA (covalently closed circular DNA). At the late stage of infection, transcription of cccDNA into RNA, encapsidation, reverse transcription, envelopment, and viral release are performed.

The NTCP inhibitor of the present invention inhibits HBV or HDV binding to NTCP and NTCP-mediated cellular entry at the initial stage of infection by inhibiting at least a portion of the functions of NTCP, and can thus be used in treatment or prevention of a disease related to HBV or HDV.

The NTCP inhibitor of the present invention inhibits at least a portion of NTCP, preferably human NTCP.

Examples of the disease related to HBV or HDV include acute hepatitis, chronic hepatitis, fulminant hepatitis, liver cirrhosis, liver failure, and hepatocellular cancer.

In the present invention, the cyclic peptide may be used as a pharmaceutically acceptable salt. Examples of the pharmaceutically acceptable salt of the cyclic peptide include salts with pharmaceutically acceptable bases or acids.

Specific examples of the pharmaceutically acceptable salt include addition salts of inorganic acids (hydrochloric acid, hydrobromic acid, hydroiodic acid, sulfuric acid, phosphoric acid, etc.), addition salts of organic acids (p-toluenesulfonic acid, methanesulfonic acid, oxalic acid, p-bromophenylsulfonic acid, carboxylic acid, succinic acid, citric acid, benzoic acid, acetic acid, etc.), and addition salts of inorganic bases (ammonium hydroxide or alkali or alkaline earth metal hydroxide, carbonate, bicarbonate, etc.) and amino acids.

The cyclic peptide serving as the NTCP inhibitor of the present invention is considered to allosterically inhibit, particularly, NTCP-mediated HBV or HDV entry into cells, via the Xw1-W-Xw2-W structure. Therefore, the cyclic peptide is not particularly limited as long as the cyclic peptide comprises the Xw1-W-Xw2-W structure. The cyclic peptide has an amino acid sequence that can be represented by the following general formula:

(X4)m-Xw1-W-Xw2-W-(X5)n

In the general formula, Xw1 and Xw2 are each independently T, S, I, L, or V, or a N-alkylamino acid thereof.

Xw1 is preferably T or L or a N-alkylamino acid thereof.

Xw2 is preferably T or I or a N-alkylamino acid thereof.

Examples of the N-alkylamino acid include N-methylamino acid.

X4 and X5 are each independently any amino acid, and at least one of m and n is an integer of 1 or larger. X4 and X5 form a cyclic structure, thereby forming a cyclic peptide. The cyclic structure may be formed through the binding of N-terminal X4 to C-terminal X5, may be formed through the binding of N-terminal X4 to non-C-terminal X5, may be formed through the binding of non-N-terminal X4 to C-terminal X5, or may be formed through the binding of non-N-terminal X4 to non-C-terminal X5.

Alternatively, X4 and X5 may form a cyclic structure via a linker.

When m is 0, Xw1 is bound to X5. When n is 0, X4 is bound to W.

m and n are not particularly limited as long as m + n is 1 or larger. m and n are preferably each independently selected integers such that m + n is 1 or larger and 26 or smaller. m and n are appropriately selected within the range of 1 or larger and 26 or smaller such that the number of amino acids constituting the cyclic structure falls within the given range.

The cyclic peptide may have a Xw1-W-Xw2-W-Xw3-W structure.

Xw3 is T, S, I, L, or V, or a N-alkylamino acid thereof.

Xw3 is preferably T or I or a N-alkylamino acid thereof.

The cyclic peptide serving as the NTCP inhibitor of the present invention is considered to allosterically inhibit, particularly, NTCP-mediated HBV or HDV entry into cells, via the P-Xp1-Xp2-H structure. Therefore, the cyclic peptide is not particularly limited as long as the cyclic peptide comprises the P-Xp1-Xp2-H structure. The cyclic peptide has an amino acid sequence that can be represented by the following general formula:

(X4)m-P-Xp1-Xp2-H-(X5)n

In the general formula, Xp1 is any amino acid, and Xp2 is I, L, or V, or a N-alkylamino acid thereof.

Xp2 is preferably I or L or a N-alkylamino acid thereof.

X4 and X5 are each independently any amino acid, and at least one of m and n is an integer of 1 or larger. X4 and X5 form a cyclic structure, thereby forming a cyclic peptide. The cyclic structure may be formed through the binding of N-terminal X4 to C-terminal X5, may be formed through the binding of N-terminal X4 to non-C-terminal X5, may be formed through the binding of non-N-terminal X4 to C-terminal X5, or may be formed through the binding of non-N-terminal X4 to non-C-terminal X5.

Alternatively, X4 and X5 may form a cyclic structure via a linker.

When m is 0, Xp1 is bound to X5. When n is 0, X4 is bound to H.

m and n are not particularly limited as long as m + n is 1 or larger. m and n are preferably each independently selected integers such that m + n is 1 or larger and 26 or smaller. m and n are appropriately selected within the m + n range of 1 or larger and 26 or smaller such that the number of amino acids constituting the cyclic structure falls within the given range.

The cyclic peptide may have a P-Xp1-Xp2-H-Xp3-F structure.

Xp3 is I, L, or V, or a N-alkylamino acid thereof.

Xp3 is preferably I or L or a N-alkylamino acid thereof.

The cyclic peptide may form a cyclic structure through a N-CO-CH₂-S structure. This structure is preferably formed through the binding of an amino group of N-terminal X4 to an acetyl group with H substituted by a leaving group, such as a chloroacetyl group, and a thiol group of cysteine of C-terminal X5. In this case, the general formula is preferably represented by XCH₂CO-(X6)-(X7)p-Xwl-W-Xw2-W-(X8)q-Cys-(X9)r, or XCH₂CO-(X6)-(X7)p-P-Xp1-Xp2-H-(X8)q-Cys-(X9)r.

X6 to X9 are each independently any amino acid, and p and q are appropriately selected, preferably within the p + q range of 0 or larger and 24 or smaller, such that the number of amino acids constituting the cyclic structure falls within the given range.

X6 is preferably Trp, and C-terminal X9 is preferably Gly or Ser. r is not particularly limited as long as r is an integer of 0 or larger. r may be 20 or smaller or may be 10 or smaller and is preferably 0 or 1.

In the present specification, when r is 0, the cyclic peptide does not have X9. When r is an integer of 2 or larger, (X9)r in the cyclic peptide corresponds to a chain-like structure where amino acids are connected through a peptide bond.

The cyclic peptide having a Xw1-W-Xw2-W-Xw3-W structure is preferably represented by the general formula XCH₂CO-(X6)-(X10)s-Xw1-W-Xw2-W-Xw3-W-(X11)t-Cys-(X9)r,
more preferably XCH₂CO-Trp-(X10)s-Xw1-W-Xw2-W-Xw3-W-(X11)t-Cys-(X9)r.

In this context, the cyclic structure is formed through a N-CO-CH₂-S structure by the binding of N-terminal X6 or the -CO-CH₂-X group bound to an amino group of Trp to a thiol group of Cys.

X6 and X9 to X11 are each independently any amino acid, and s and t are appropriately selected, preferably within the s + t range of 0 or larger and 22 or smaller, such that the number of amino acids constituting the cyclic structure falls within the given range.

X6 is preferably Trp, and C-terminal X9 is preferably Gys or Ser. r is not particularly limited as long as r is an integer of 0 or larger. r may be 20 or smaller or may be 10 or smaller and is preferably 0 or 1.

The cyclic peptide having a P-Xp1-Xp2-H-Xp3-F structure is preferably represented by the general formula XCH₂CO-(X6)-(X10)s-P-Xp1-Xp2-H-Xp3-F-(X12)u-Cys-(X9)r, more preferably XCH₂CO-Trp-(X10)s-P-Xp1-Xp2-H-Xp3-F-(X12)u-Cys-(X9)r.

In this context, the cyclic structure is formed through a N-CO-CH₂-S structure by the binding of N-terminal X6 or the -CO-CH₂-X group bound to an amino group of Trp to a thiol group of Cys.

X6, X9, X10 and X12 are each independently any amino acid, and s and u are appropriately selected, preferably within the s + u range of 0 or larger and 22 or smaller, such that the number of amino acids constituting the cyclic structure falls within the given range.

X6 is preferably Trp, and C-terminal X9 is preferably Gly or Ser. r is not particularly limited as long as r is an integer of 0 or larger. r may be 20 or smaller or may be 10 or smaller and is preferably 0 or 1.

Specific examples of the cyclic peptide of the present invention include the following:

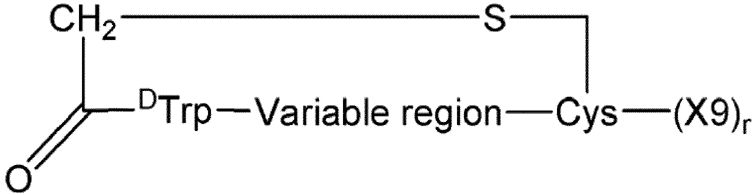

In the structure, ^{D}Trp represents a D form of Trp, and S represents a thiol group of Cys. X9 and r are as defined above.

Examples of the Variable region in the formula include
ALWIWPQVYWTRFS
WWAINPHIHVFTWP
ILVYPELHLFLLYS
LYTWTWHSNWTVTSL
LYLWTWPNTSYAYNV
SYIDTFTWIWLY
FIIV^{Me}GTWTWTWS
TWIW^{Me}G^{Me}GLYWYFP
ALIWTW^{Me}SQYYQY^{Me}Y_{Me}^{Me}S
TFTY^{Me}AQIWYY^{Me}G
TYKYS^{Me}GIWIWIW
IWIWPGYVIY
TWTWH^{Me}SAYLYVF
VIWIWPNHFYY
TWTWQNSFIWIY
TWTWN^{Me}SAFLYVY
NYIYYP^{Me}SIHIFYL, and
HWFYP^{Me}SIHVFTW^{Me}A.

The cyclic peptide may comprise an amino acid sequence derived from any of these amino acid sequences by addition, substitution, or deletion of 1 or several, preferably 1 to 6, 1 to 5, 1 to 4, 1 to 3, or 1 or 2 amino acids and have an effect of inhibiting HBV or HDV entry into cells via NTCP, as long as the cyclic peptide has a Xw1-W-Xw2-W structure or a P-Xp1-Xp2-H structure and preferably has a Xw1-W-Xw2-W-Xw3-W structure or preferably has a P-Xp1-Xp2-H-Xp3-F structure.

Also, the cyclic peptide may comprise an amino acid sequence having 70% or higher, 80% or higher, 90% or higher, 95% or higher, or 98% or higher sequence identity to any of these amino acid sequences and have an effect of inhibiting HBV or HDV entry into cells via NTCP, as long as the cyclic peptide has a Xw1-W-Xw2-W structure or a P-Xp1-Xp2-H structure and preferably has a Xw1-W-Xw2-W-Xw3-W structure or preferably has a P-Xp1-Xp2-H-Xp3-F structure.

The cyclic peptide of the present invention can be suitably produced by a translation and synthesis method using a cell-free translation system.

A nucleic acid encoding the peptide to be cyclized is prepared, and this nucleic acid can be translated in a cell-free translation system to prepare the cyclic peptide. The nucleic acid encoding the peptide to be cyclized can be appropriately designed by those skilled in the art using genetic codes for use in translation systems of organisms, reprogrammed genetic codes, or a combination thereof. The nucleic acid may be DNA or may be RNA.

According to a method using the cell-free translation system, natural amino acids as well as non-natural amino acids can be efficiently introduced to the peptide using tRNA aminoacylated with non-natural amino acids. For example, use of an artificial aminoacylated tRNA-synthesizing enzyme flexizyme developed by the present inventors permits aminoacylation of tRNA having any anticodon with any natural or non-natural amino acid. Thus, this technique can be used to reprogram genetic codes consisting of mRNA triplets so as to encode amino acids different from those of translation systems of organisms (International Publication No. WO 2008/059823).

The NTCP inhibitor of the present invention can be used as a pharmaceutical composition comprising the same.

The dosage form of the pharmaceutical composition is not particularly limited and may be oral administration or may be parenteral administration. Examples of the parenteral administration include administration by injection such as intramuscular injection, intravenous injection, and subcutaneous injection, percutaneous administration, and transmucosal administration.

Examples of the administration route of the transmucosal administration include transnasal, transoral, transocular, transpulmonary, transvaginal, and transrectal routes.

The cyclic peptide in the pharmaceutical composition may be variously modified from the viewpoint of pharmacokinetics such as metabolism or excretion. For example, polyethylene glycol (PEG) or a sugar chain can be added to the cyclic peptide to prolong a retention time in blood and reduce antigenicity.

A sustained-release base such as a degradable polymer compound (e.g., poly-lactide-co-glycolide acid (PLGA)), porous hydroxyapatite, a liposome, a surface-modified liposome, an emulsion prepared from an unsaturated fatty acid, nanoparticles, or a nanosphere is used, and the cyclic peptide may be enclosed therein. For percutaneous administration, the stratum corneum may be permeated by flowing weak current through skin surface (iontophoresis method).

In the pharmaceutical composition, the cyclic peptide may be used directly as an active ingredient, or may be formulated into a preparation by the addition of pharmaceutically acceptable additives or the like.

Examples of the dosage form of the pharmaceutical preparation include solutions (e.g., injections), dispersions, suspensions, tablets, powders, suppositories, dusts, fine granules, granules, capsules, syrups, troches, inhalants, ointments, eye drops, nasal drops, ear drops, and poultices.

The formulation can be performed by a routine method appropriately using additives, for example, an excipient, a binder, a disintegrant, a lubricant, a solubilizer, a dissolution aid, a colorant, a corrigent, a stabilizer, an emulsifier, an absorption promoter, a surfactant, a pH adjuster, an antiseptic, a wetting agent, a dispersant, and an antioxidant.

Examples of the additives for use in the formulation include, but are not particularly limited to, purified water, saline, phosphate buffer solutions, dextrose, glycerol, pharmaceutically acceptable organic solvents such as ethanol, animal or plant oils, lactose, mannitol, glucose, sorbitol, crystalline cellulose, hydroxypropylcellulose, starch, corn starch, silicic anhydride, aluminum magnesium silicate, collagen, polyvinyl alcohol, polyvinylpyrrolidone, carboxyvinyl polymers, sodium carboxymethylcellulose, sodium polyacrylate, sodium alginate, water-soluble dextran, sodium carboxymethyl starch, pectin, methylcellulose, ethylcellulose, xanthan gum, gum arabic, tragacanth, casein, agar, polyethylene glycol, diglycerin, glycerin, propylene glycol, petrolatum, paraffin, octyldodecyl myristate, isopropyl myristate, higher alcohol, stearyl alcohol, stearic acid, and human serum albumin.

An absorption promoter including: surfactants such as polyoxyethylene lauryl ethers, sodium lauryl sulfate, and saponin; salts of bile acids such as glycocholic acid, deoxycholic acid, and taurocholic acid; chelating agent such as EDTA and salicylic acid; fatty acids such as caproic acid, capric acid, lauric acid, oleic acid, linoleic acid, and mixed micelle; and enamine derivatives, N-acyl collagen peptides, N-acylamino acids, cyclodextrins, chitosans, and nitrogen monoxide donors may be used for transmucosal absorption.

Tablets or pills may be coated tablets or the like having a coating with s sugar coating, gastric coating, or enteric coating material, etc.

Solution may contain injectable distilled water, physiological saline, propylene glycol, polyethylene glycol, plant oil, and alcohols, etc. A wetting agent, an emulsifier, a dispersant, a stabilizer, a solubilizer, a dissolution aid, and an antiseptic, etc. may be added thereto.

The present invention also provides a method for treating or preventing a disease in a patient, comprising administering the NTCP inhibitor to the patient in need thereof.

The dose of the NTCP inhibitor of the present invention can be appropriately determined by those skilled in the art according to the symptoms, age, sex, body weight, and sensitivity difference of the patient in need thereof, an administration method, administration intervals, and the type of a preparation, etc.

The patient is a mammal, preferably a human.

### Examples

Hereinafter, the present invention will be described with reference to Examples. However, the present invention is not limited by Examples given below.

### <Material and method>

### Material

Fmoc-protected amino acids were purchased from Merck Millipore. Oligonucleotides were purchased from Eurofins Genomics K.K. (Japan). Other reagents were obtained from Nacalai Tesque, Inc. unless otherwise specified.

### RaPID selection

Cyanomethyl ester (CME)- or dinitrobenzyl ester (DBE)-activated amino acids were synthesized by a known method (the method described in Morimoto, J., Hayashi, Y. & Suga, H. Discovery of Macrocyclic Peptides Armed with a Mechanism-Based Warhead: Isoform-Selective Inhibition of Human Deacetylase SIRT2. Angew. Chem. Int. Ed. Engl. 51, 3423-3427 (2012), and Kawakami, T., Murakami, H. & Suga, H. Messenger RNA-programmed incorporation of multiple N-methyl-amino acids into linear and cyclic peptides. Chem. Biol. 15, 32-42 (2008)). Flexizyme aminoacylating ribozyme and tRNA were prepared by *in vitro* transcription from PCR generated DNA templates (Table 1 described below) and by tRNA aminoacylation using CMC- or DBE-activated amino acids (Goto, Y., Katoh, T. & Suga, H. Flexizymes for genetic code reprogramming. Nat. Protoc. 6, 779-790 (2011)). Specifically, RNA molecules were synthesized through T7 RNA polymerase reaction from DNA templates assembled by PCR (Table 1), and purified by PAGE. 40 mM each tRNA substrate and flexizyme aminoacylating ribozyme, 600 mM MgCl₂, 5 mM activated amino acid substrate and 100 mM HEPES-KOH (pH 7.5) were added on ice for 2 hours (N-chloroacetyl-tyrosine-CME, N-methyl-glycine-DBE, N-methyl-alanine-DBE) or 6 hours (N-methyl-4-O-methyl-tyrosine-CME, N-methyl-serine-DBE, N-methyl-phenylalanine-CME and N-methyl-leucine-DBE), followed by recovery by precipitation with sodium acetate and ethanol. Puromycin-bound oligonucleotides (Table 1) and T4 RNA ligase were purified by phenol:chloroform extraction and ethanol precipitation to prepare puromycin-bound mRNA.

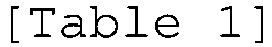

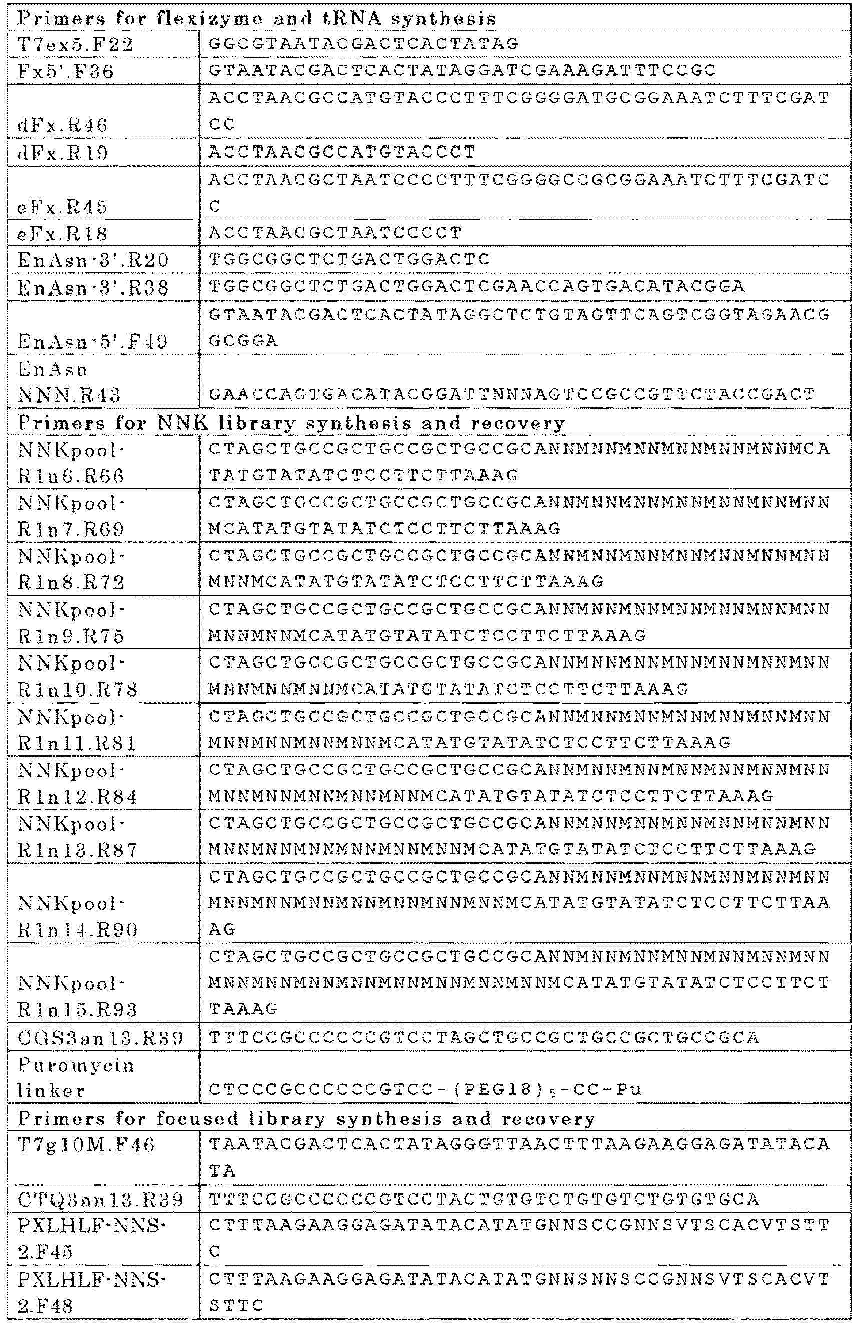

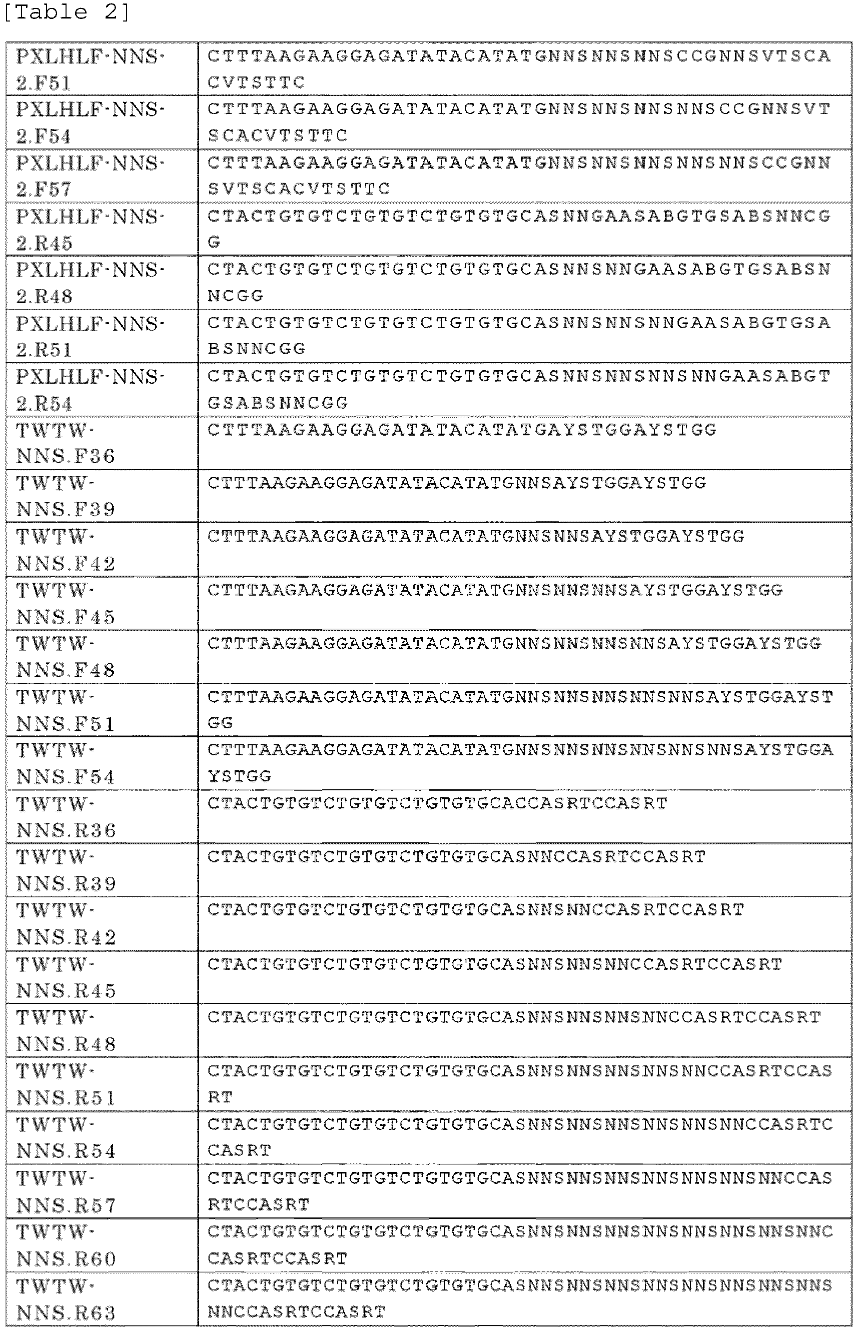

Ribosomes for cyclic peptide libraries were synthesized by a known method (the method described in Hayashi, Y., Morimoto, J. & Suga, H. In vitro selection of anti-Akt2 thioether-macrocyclic peptides leading to isoform-selective inhibitors. ACS Chem. Biol. 7, 607-613 (2012)). Specifically, for the first selection, 1.2 µM puromycin-bound mRNA library was translated in a Met-deficient FIT reaction containing 25 µM L- or D-ClAc-Tyr-tRNAfMet at 37°C for 30 minutes. The resulting product was incubated at 25°C for 12 minutes, followed by disruption of the ribosome-mRNA complex by incubation at 37°C for 30 minutes in the presence of 20 mM EDTA. Subsequently, the obtained peptide-bound mRNA was reverse transcribed using RNase H-reverse transcriptase (Promega) at 42°C for 1 hour, and the buffer solution was exchanged for 50 mM Tris-HCl (pH 7.4), 150 mM NaCl, 0.05 vol% Tween-20 and 1 mM βDDM. Six serial passages (counter selections at 4°C for 30 min each) over His pull-down Dynabeads (trade name of Life Technologies Corp.) were performed, and subsequent affinity selection against 200 nM NTCP immobilized on the same beads was performed at 4°C for 30 minutes. cDNA was eluted from the beads by heating to 95°C for 5 minutes, and fractional recovery from the final counter selection (negative control) and the affinity selection step was evaluated by quantitative PCR using Sybr Green I on a LightCycler thermal cycler (F. Hoffmann-La Roche, Ltd.). Enriched DNA libraries were recovered by PCR, and used as input for transcription reaction to generate a mRNA library for the next round of screening. For focused selections mentioned later, the selection was performed except that the following FIT reaction: the FIT reaction was deficient in Met, Asp, Glu, and Arg as well as their cognate aminoacyl tRNA synthetases, and contained 6.25 µM L- or D-ClAc-Tyr-tRNAfMet and 12.5 µM each of EnGluGUC-MeGly, EnGluCUC-MeAla, EnGluGCG-MeTyr(Me), EnGluCCG-MePhe and EnGluCCU-MeSer.

For sequencing of the first selections, DNA samples were cloned into the pGEM-T-easy vector (Promega Corp.) using DNA Ligation Kit Mighty Mix (Takara Bio Inc.), and competent DH5a *E. Coli* was transformed with the vector. 24 clones were sequenced from each sample. For high-throughput sequencing of the focused selections, DNA samples were amplified by PCR with nesting primers (Table 1), purified using a Nucleospin column (Macherey-Nagel GmbH & Co. KG) and sequenced using a MiSeq high-throughput sequencer (Illumina, Inc.). Data analysis was conducted using CLC workbench software (Qiagen N.V.).

### Solid phase peptide synthesis

Cyclic peptides were synthesized by standard Fmoc solid phase synthesis using a Syro Wave automated synthesizer. For synthesis of N-methyl-O-methyl-tyrosine residues, O-methyl-tyrosine coupling was followed by Fmoc deprotection, Nosyl protection of the obtained free amine, and on-resin methylation using dimethyl sulfate as follows: after Fmoc deprotection, nitrobenzenesulfonyl chloride (22 mg, 0.1 mmol) in N-methyl-2-pyrrolidone (NMP) and 2,4,6-trimethylpyridine (33 µL, 0.25 mmol) was added to the resin so that the reaction was allowed to proceed for 15 minutes. The resin was washed 5 times with NMP at room temperature. Methylation was performed twice using 1,8-diazabicyclo(5.4.0)undec-7-ene (11.25 µL, 0.075 mmol) and dimethyl sulfate (22.25 µL, 0.25 mmol) in NMP for 5 minutes. Then, the resin was washed 5 times) exchanged for NMP. Nosyl deprotection was performed twice using 1,8-diazabicyclo(5.4.0)undec-7-ene (18.75 µL, 0.125 mmol) and 2-mercaptoethanol (17.5 µL, 0.25 mmol) in NMP for 5 minutes. Then, the resin was washed 5 times with NMP, and solid phase peptide synthesis was continued. After the final Fmoc deprotection step, each peptide was N-terminally chloroacetylated through reaction with 8 equivalents of chloroacetyl-N-hydroxy-succinamide at room temperature for 1 hour. Cleavage from the resin and side chain deprotection were performed using 2.5 vol% each triisopropylsilane, ethanedithiol and water in trifluoracetic acid (TFA). Peptides were recovered by precipitation with diethyl ether dissolved in dimethyl sulfoxide (DMSO), and rendered basic with N,N,N-triethylamine for cyclization. Purification was performed on a Prominence HPLC system (Shimadzu Corp.) using a water:acetonitrile gradient (containing 0.1% TFA). The final TFA salt was freeze-dried 3 times from 5 mM aqueous HCl solution to obtain the HCl salt, which was then dissolved in DMSO and purified for the next studies.

### Surface plasmon resonance

The association constant of each peptide for human NTCP was measured using a Biacore T200 apparatus (GE Healthcare Japan Corp.). The running buffer solution used was HBS-P+ (10 mM HEPES, 150 mM NaCl, and 0.05 vol% surfactant P20, pH 7.4) containing 0.1 vol% DMSO and 1 mM β-dodecyl-maltoside. 100 nM recombinant NTCP was immobilized on a NTA chip treated with Ni²⁺ according to the manufacturer's instructions (baseline response: 1000 to 3000 refraction units). Peptides were injected as analytes at a flow rate of 60 µL/min, and association constants were modelled using a 1:1 binding model.

### Preparation of recombinant NTCP protein

His-tagged recombinant NTCP protein was synthesized using a wheat germ cell-free protein synthesis system in a known method (the method described in Watashi, K. et al. Cyclosporin A and its analogs inhibit hepatitis B virus entry into cultured hepatocytes through targeting a membrane transporter, sodium taurocholate cotransporting polypeptide (NTCP). Hepatology 59, 1726-1737 (2014), and Shimura, S. et al. Cyclosporin derivatives inhibit hepatitis B virus entry without interfering with NTCP transporter activity. Journal of hepatology 66, 685-692 (2017)). The structure and functions of the synthesized NTCP protein were confirmed by the following 3 methods (the methods described in Watashi, K. et al. Cyclosporin A and its analogs inhibit hepatitis B virus entry into cultured hepatocytes through targeting a membrane transporter, sodium taurocholate cotransporting polypeptide (NTCP). Hepatology 59, 1726-1737 (2014)).
1) The NTCP protein interacted with [³H]-taurocholic acid in a scintillation proximity assay.
2) The NTCP protein specifically interacted with HBV-L surface antigen, and this interaction was dissociated in the presence of an excess of preS1 peptide, as examined by alpha screen assay.
3) Addition of the NTCP protein during HBV infection cell culture assay significantly inhibited HBV infection of cells.

### Cell culture

HepG2-hNTCP-C4, Hep38.7-Tet, HepG2, and Huh-7.5.1 (provided by Dr. Francis Chisari of Scripps Research Institute) cells and primary human hepatocytes (PhoenixBio Co., Ltd.) were cultured as described in known methods (the methods described in Iwamoto, M. et al. Evaluation and identification of hepatitis B virus entry inhibitors using HepG2 cells overexpressing a membrane transporter NTCP. Biochemical and biophysical research communications 443, 808-813 (2014)., Ogura, N., Watashi, K., Noguchi, T. & Wakita, T. Formation of covalently closed circular DNA in Hep38.7-Tet cells, a tetracycline inducible hepatitis B virus expression cell line. Biochemical and biophysical research communications 452, 315-321 (2014), and Ishida, Y. et al. Novel robust in vitro hepatitis B virus infection model using fresh human hepatocytes isolated from humanized mice. The American journal of pathology 185, 1275-1285 (2015)).

### HBV preparation and infection

HBV genotype D used in this study (Figures 1 to 3) was derived from the culture supernatant of Hep38.7-Tet cells, and prepared by a known method (the method described in Tsukuda, S. et al. Dysregulation of retinoic acid receptor diminishes hepatocyte permissiveness to hepatitis B virus infection through modulation of sodium taurocholate cotransporting polypeptide (NTCP) expression. The Journal of biological chemistry 290, 5673-5684 (2015), and Ogura, N., Watashi, K., Noguchi, T. & Wakita, T. Formation of covalently closed circular DNA in Hep38.7-Tet cells, a tetracycline inducible hepatitis B virus expression cell line. Biochemical and biophysical research communications 452, 315-321 (2014)). Purified HBV (Figure 1(d)) was prepared by ultrafiltration to remove an excess of components in FBS and medium. HBV genotypes A, B, and C and genotype C having the L180M/S202G/M204V mutations (nucleoside analog-resistant HBV) or G145R substitution (vaccine-escape HBV) (Figure 4(d)) were prepared from the culture supernatant of HepG2 cells transfected with the corresponding expression plasmid. HBV was inoculated at 12,000 (Figures 1(a), 1(b), and 1(d)), 1000 to 2000 (Figure 2(b)) or 500 (Figures 3(a) and 4(d)) genome equivalents (GEq)/cell in the presence of 4% polyethylene glycol 8000 (PEG8000) and incubated at 37°C for 16 hours by a known method (the method described in Watashi, K. et al. Interleukin-1 and tumor necrosis factor-alpha trigger restriction of hepatitis B virus infection via a cytidine deaminase activation-induced cytidine deaminase (AID). The Journal of biological chemistry 288, 31715-31727 (2013)).

### Detection of HBs and HBe antigens

HBe antigen was detected by a chemiluminescence immunoassay (CLIA) (LSI Medience Corp.). HBs antigen was quantified by enzyme-linked immunosorbent assay (ELISA) as described in a known method (the method described in Watashi, K. et al. Interleukin-1 and tumor necrosis factor-alpha trigger restriction of hepatitis B virus infection via a cytidine deaminase activation-induced cytidine deaminase (AID). The Journal of biological chemistry 288, 31715-31727 (2013)).

### MTT assay

3-(4,5-Dimethylthial-2-yl)-2,5-diphenyltetrazolium bromide (MTT) assay for measurement of cell viability was conducted as described in a known method (the method described in Watashi, K. et al. Interleukin-1 and tumor necrosis factor-alpha trigger restriction of hepatitis B virus infection via a cytidine deaminase activation-induced cytidine deaminase (AID). The Journal of biological chemistry 288, 31715-31727 (2013)).

### Indirect immunofluorescence analysis

Immunofluorescence analysis was conducted using anti-HBc antibody (Thermo Fisher Scientific, Waltham, MA, RB-1413-A) at a dilution of 1:100 as described in a known method (the method described in Watashi, K. et al. Interleukin-1 and tumor necrosis factor-alpha trigger restriction of hepatitis B virus infection via a cytidine deaminase activation-induced cytidine deaminase (AID). The Journal of biological chemistry 288, 31715-31727 (2013)).

### HBV replication assay

3 days after cell seeding in the presence of tetracycline, Hep38.7-Tet cells were treated with peptides in the absence of tetracycline for 6 days. HBV DNA released into a culture supernatant was recovered, and quantified by real-time PCR as described in a known method (the method described in Ogura, N., Watashi, K., Noguchi, T. & Wakita, T. Formation of covalently closed circular DNA in Hep38.7-Tet cells, a tetracycline inducible hepatitis B virus expression cell line. Biochemical and biophysical research communications 452, 315-321 (2014)).

### Real-time PCR

Real-time PCR for quantification of HBV DNA was performed as described in a known method (the method described in Watashi, K. et al. Interleukin-1 and tumor necrosis factor-alpha trigger restriction of hepatitis B virus infection via a cytidine deaminase activation-induced cytidine deaminase (AID). The Journal of biological chemistry 288, 31715-31727 (2013)).

### PreS1 binding assay

The interaction between preS1 peptide and host cells was evaluated by incubating HepG2-hNTCP-C4 cells with 40 nM 6-carboxytetramethylrhodamine (TAMRA)-labeled preS1 peptide (preS1 probe) at 37°C for 30 minutes. The cells were washed, fixed, and nuclearly stained with 4,6-diamidino-2-phenylindole (DAPI) as described in a known method (the method described in Kaneko, M. et al. A Novel Tricyclic Polyketide, Vanitaracin A, Specifically Inhibits the Entry of Hepatitis B and D Viruses by Targeting Sodium Taurocholate Cotransporting Polypeptide. Journal of virology 89, 11945-11953 (2015)).

### NTCP transporter assay

HepG2-hNTCP-C4 cells pretreated with peptides for 30 minutes were incubated with [³H]-taurocholic acid (TCA) in the presence of the peptides at 37°C for 15 minutes so that the cells took up TCA. After washing of free [³H]-TCA, the cells were lysed, and intracellular radioactivity was measured by a known method (the method described in Kaneko, M. et al. A Novel Tricyclic Polyketide, Vanitaracin A, Specifically Inhibits the Entry of Hepatitis B and D Viruses by Targeting Sodium Taurocholate Cotransporting Polypeptide. Journal of virology 89, 11945-11953 (2015)).

### HDV infection assay

HDV was prepared from the culture supernatant of Huh-7 cells transfected with an expression plasmid as described in a known method (the method described in Kaneko, M. et al. A Novel Tricyclic Polyketide, Vanitaracin A, Specifically Inhibits the Entry of Hepatitis B and D Viruses by Targeting Sodium Taurocholate Cotransporting Polypeptide. Journal of virology 89, 11945-11953 (2015)) (the HDV expression plasmid pSVLD3 was provided by Dr. John Taylor of Fox Chase Cancer Center). HDV was inoculated at 5 GEq/cell in the presence of 4% PEG8000 at 37°C for 16 hours (Figure 4(b)), and intracellular HDV RNA was measured to evaluate infection.

### HCV pseudoparticle assay

HCV envelope-dependent entry was evaluated using the HCV pseudoparticle system (provided by Dr. Francois-Loic Cosset of the University of Lyon) (Bartosch, B., Dubuisson, J. & Cosset, F.L. Infectious hepatitis C virus pseudo-particles containing functional E1-E2 envelope protein complexes. The Journal of experimental medicine 197, 633-642 (2003)). Huh-7.5.1 cells were pretreated with peptides for 1 hour, and inoculated with HCV pseudoparticles in the presence of the peptides for 4 hours. 72 hours after infection, luciferase activity was measured by a known method (the method described in Nakajima, S. et al. Specific inhibition of hepatitis C virus entry into host hepatocytes by fungi-derived sulochrin and its derivatives. Biochemical and biophysical research communications 440, 515-520 (2013)).

### Statistics

Statistical significance was determined using Student's t-test (*p < 0.05, **p < 0.01, N.S.: not significant).

### <Results>

### Initial RaPID selection against NTCP

In order to study the inhibition of HBV cellular entry by cyclic peptides, selection based on the RaPID system was carried out using purified His-tagged NTCP (the structure and functions of which can be confirmed, at least in part, from the description of Watashi, K. et al. Cyclosporin A and its analogs inhibit hepatitis B virus entry into cultured hepatocytes through targeting a membrane transporter, sodium taurocholate cotransporting polypeptide (NTCP). Hepatology 59, 1726-1737 (2014) and the aforementioned section <Material and method>) as a target to obtain a peptide library. The obtained peptide library was generated by transcription and translation from a semi-randomized DNA template containing an AUG start codon, a randomized region of 4 to 15 sequential NNK codons (N = A, G, C or T, and K = T or G), a Cys codon, and a sequence encoding a triple-repeat of Gly-Ser linker (Figure 5(b)). A genetically reprogrammed translation system was used in which initiating formyl-Met of each peptide was replaced with L- or D-N-chloroacetylated-Tyr, which was then reacted with downstream Cys to form a thioether bridged cyclic structure (Figure 5(b)). In this way, two libraries (one L-initiated and one D-initiated) each containing more than 10¹² types of cyclic peptides were prepared and screened.

After seven rounds of selection, sequence analysis of enriched DNA libraries from after the fifth, sixth, and seventh rounds revealed that ten peptides were common or homologous (six from the L-library and four from the D-library; Table 2 described below). Among them, three (NTCP-D1, -D4, and -L4) containing internal Met codons underwent replacement of these Met residues with Ile/Leu and/or Thr because Met was removed from the translation system. This was confirmed by measuring the specific peptides translated in the FIT system by MALDI-TOF MS (Figure 6). Analysis of the affinity for NTCP of individual clones of these peptides mutated to involve Ile, Leu, or Thr codons in place of the Met codon employed the M5I, M12L, and M10T variants in active forms of NTCP-D1, NTCP-D4, and NTCP-L4, respectively, though only minor variations in binding affinity were observed (Figure 6).

**[Table 3]**

| | | | K_{D} (nM) |
|---|---|---|---|
| Initial screening | **D1** | -**S-Ac**-D-YALNIWPQVYNTRFSCG-**NH₂** | 1340 |
| | **D2** | -**S-Ac**-D-YWWAINPHIHVFTWPCG-**NH₂** | 14 |
| | D3 | -**S-Ac**-D-YHYWYPENNTALYIWPCG-**NH₂** | 50 |
| | D4 | -**S-Ac**-D-YILVYPELHLFLLYSCG-**NH₂** | 2270 |
| | L1 | -**S-Ac**-YLYTWTWHSNWTVTSLCG-**NH₂** | 21 |
| | L2 | -**S-Ac**-YVWLLWAERSWFLDLGCG-**NH₂** | 374 |
| | L3 | -**S-Ac**-YLWIFHQNWYTFDLGCG-**NH₂** | 13 |
| | L4 | -**S-Ac**-YLYLWTWPNTSYAYNVCG-**NH₂** | 30 |
| | L5 | -**S-Ac**-YTWNTHHYYIWLNCG-**NH₂** | 4 |
| | L6 | -**S-Ac**-YSYIDTFTWIWLYCG-**NH₂** | 36 |
| Focused screening | **WD1** | -**S-Ac**-D-YFIIV^{Me}GTWTWTWSCT-**NH₂** | 15 |
| | WD2 | -**S-Ac**-D-YTWIW^{Me}G^{Me}GLYVVYFPCT-**NH₂** | 14 |
| | WD3 | -**S-Ac**-D-YALIWTW^{Me}SQYYQY^{Me}Y_{Me}^{Me}SCT-**NH₂** | 15 |
| | WD4 | -**S-Ac**-D-YTFTY^{Me}AQIWIWYY^{Me}GCT-**NH₂** | 11 |
| | WL1 | -**S-Ac**-YTYKYS^{Me}GIWIWIWCT-**NH₂** | 360 |
| | **WL2** | -**S-Ac**-YIWIWPGYVIYCT-**NH₂** | 157 |
| | WL3 | -**S-Ac**-YTWTWH^{Me}SAYLYVFCT-**NH₂** | 57 |
| | **WL4** | -**S-Ac**-YVIWIWPNHFYYCT-**NH₂** | 42 |
| | WL5 | -**S-Ac**-YTWTWQNSFIWIYCT-**NH₂** | 10 |
| | WL6 | -**S-Ac**-YTWTWN^{Me}SAFLYVYCT-**NH₂** | 36 |
| | PD1 | -**S-Ac**-D-YNYIYYP^{Me}SIHIFYLCT-**NH₂** | 149 |
| | PL1 | -**S-Ac**-YHWFYP^{Me}SIHVFTW^{Me}ACT-**NH₂** | 228 |

In order to further characterize the identified cyclic peptides, the 10 peptides shown in Table 2 were synthesized by solid phase peptide methodology without the (GS)₃ inker sequence (and with active forms of the Met mutations for each of NTCP-D1, -D4, and -L4). The respective association constants of these peptides toward NTCP were evaluated by surface plasmon resonance (Table 2; Figure 7). Some peptides (NTCP-D2, -D3, -L1, -L3, - L4, -L5, and -L6) exhibited strong binding with a dissociation constant (KD) of 4 to 50 nM while 3 peptides exhibited weaker binding with a KD value of greater than 100 nM.

Subsequently, the present inventors evaluated the effect of the 10 peptides described above on HBV infection in primary human hepatocytes. In these assays, cells were pretreated with the peptides for 2 hours, subsequently inoculated with HBV in the presence of the peptides for 16 hours, then washed, and further cultured for 12 days in the absence of the peptides (Figure 2(a)). HBV infection was evaluated by measuring HBs antigen in the culture supernatant. CsA, a natural cyclic peptide inhibiting HBV entry by binding to NTCP, was used as a positive control (Figure 2(b)). In all cases, treatment with each peptide at 100 µM significantly reduced HBs antigen levels (Figure 2(b)) with no marked cytotoxic effects (Figure 2(c)). This indicates that all of the peptides, including the newly discovered thioether-macrocycles, have anti-HBV activity.

### Focused library RaPID selection

During inspection of the sequences of the peptides from the first round of screening and activity studies, the present inventors confirmed that two of the most active peptides in the HBV infectivity assay, NTCP-D2 and -D4, contained a PX(I/L)H(I(T/I)W(T/I)W motif (hereinafter, referred to as the "W motif)", and three others, NTCP-L1, -L4, and -D1, contained a (T/L)W(T/I)P motif (referred to as the "P motif"). On the basis of this observation, FIT translation was used to construct 4 focused peptide libraries (two L-ClAc-Tyr initiated and two D-ClAc-Tyr initiated) based on the P or W motif (Figure 8). These libraries were generated using reprogrammed genetic codes in which Met, Asp, Glu, and Arg were replaced with N-methyl-Leu, Ser, Gly, Ala and Phe, and N-methyl-O-methyl-Tyr. These libraries can be used in order to improve the "drug-likeness" (e.g., better peptidase resistance and octanol-water partition coefficient) of the identified peptides. After seven rounds of RaPID selection with the libraries using NTCP as bait, a DNA library obtained from each round of selection was analyzed using a high-throughput sequencer. Alignment of the peptide sequences identified from the final round of selection demonstrated strong enrichment for homologous sequences in all of the 4 libraries. From these libraries, 12 peptides were selected, on the basis of relative enrichment and sequence diversity, for chemical synthesis and subsequent characterization (WD1-4, WL1-6, PD1 and PL1 in Table 2). The respective binding kinetics of these peptides toward NTCP were evaluated by surface plasmon resonance (Table 2 and Figure 9), and mostly exhibited a KD value in the range of 10 to 100 nM.

Next, the present inventors evaluated the influence of the 12 peptides identified from focused library RaPID selection, in addition to the peptides from the first RaPID selection containing the W and P motifs of interest, on HBV infection of primary human hepatocytes at a concentration of 10 µM (see the description above). As shown in Figure 3, 10 out of the 12 cyclic peptides identified by focused screening significantly reduced HBV antigens, seven of which exhibited greater potency than that of the peptides identified during initial screening. In particular, the WD1, WL2, and WL4 peptides most markedly reduced HBV antigens (Figure 3(a)) without exhibiting significant cytotoxic effects (Figure 3(b)).

### Cyclic peptide having higher anti-HBV activity than that of cyclosporin A

In order to evaluate the anti-HBV activity of the 3 peptides, WD1, WL2, and WL4, HBV infection assay was performed by a known method (the method described in Iwamoto, M. et al. Evaluation and identification of hepatitis B virus entry inhibitors using HepG2 cells overexpressing a membrane transporter NTCP. Biochemical and biophysical research communications 443, 808-813 (2014)) using HepG2-hNTCP-C4 cells, which overexpress the human NTCP gene and are susceptible to HBV infection. After HBV inoculation, HBs antigen was measured in a culture supernatant, and HBc antigen was evaluated in the cells to confirm HBV infection, as shown in Figure 2(a). In this assay, CsA reduced HBV infection monitored by HBs and HBc antigens, whereas entecavir, a nucleoside analog known as an HBV replication inhibitor, did not exhibit a significant reduction. Treatment with WD1, WL2, and WL4 decreased intracellular HBc levels (Figure 1(b)) and HBs levels in the supernatant (Figure 1(c)) without marked cytotoxicity (Figure 1(a)). In particular, the anti-HBV effect of the WD1, WL2 and WL4 peptides was higher than that of D1 and D2 obtained from the first screening.

In order to evaluate anti-HBV activity under more physiological conditions, HBV infection assay was performed using primary human hepatocytes, and purified HBV was used as an inoculum (see the section <Material and method>). Treatment with each peptide reduced HBs antigen levels in a culture supernatant in a dose-dependent manner (Figure 1(d)). The 50% inhibitory concentrations (IC₅₀) of WD1, WL2, WL4 and CsA in this assay were calculated to be 0.85 ± 0.01, 2.54 ± 0.11, 0.66 ± 0.03 and 3.17 ± 0.30 µM, respectively. This indicates that the thioether-macrocycles discovered in this experiment have approximately up to 5 times higher than the anti-HBV effect of CsA.

### Inhibition of HBV cellular attachment that does not influence NTCP transporter function

The present inventors next confirmed whether the observed anti-HBV effect of WD1, WL2 and WL4 cyclic peptides was due to the inhibition of viral entry into cells. The HBV life cycle consists of the early phase of infection involving attachment, entry, nuclear import, and covalently closed circular DNA (cccDNA) formation, and the late phase (HBV replication process) involving transcription, encapsidation, reverse transcription, envelopment, and viral release (Figure 10; Watashi, K. et al. Cyclosporin A and its analogs inhibit hepatitis B virus entry into cultured hepatocytes through targeting a membrane transporter, sodium taurocholate cotransporting polypeptide (NTCP). Hepatology 59, 1726-1737 (2014), and Locarnini, S. & Zoulim, F. Molecular genetics of HBV infection. Antiviral therapy 15 Suppl 3, 3-14 (2010)). In order to examine the effect of the peptides on the late phase of the HBV life cycle, Hep38.7-Tet cells were used, which reproduce HBV replication in the absence of tetracycline but do not cause the early phase of infection due to a lack of NTCP expression. HBV replication was evaluated by quantifying intracellular HBV DNA after tetracycline withdrawal in the presence or absence of the peptides for 6 days. Under these conditions, unlike entecavir, a nucleoside analog used as a positive control, the 3 cyclic peptides rarely exhibited an effect on HBV DNA levels (Figure 10(a)).

Next, viral attachment to host cells was examined. The preS1 region of the HBV-L surface antigen is essential for viral-host attachment and the following entry process (Barrera, A., Guerra, B., Notvall, L. & Lanford, R.E. Mapping of the hepatitis B virus pre-S1 domain involved in receptor recognition. Journal of virology 79, 9786-9798 (2005)., Glebe, D. et al. Mapping of the hepatitis B virus attachment site by use of infection-inhibiting preS1 lipopeptides and tupaia hepatocytes. Gastroenterology 129, 234-245 (2005)., Gripon, P., Cannie, I. & Urban, S. Efficient inhibition of hepatitis B virus infection by acylated peptides derived from the large viral surface protein. Journal of virology 79, 1613-1622 (2005)., Yan, H. et al. Sodium taurocholate cotransporting polypeptide is a functional receptor for human hepatitis B and D virus. eLife 1, e00049 (2012)., and Le Seyec, J., Chouteau, P., Cannie, I., Guguen-Guillouzo, C. & Gripon, P. Infection process of the hepatitis B virus depends on the presence of a defined sequence in the pre-S1 domain. Journal of virology 73, 2052-2057 (1999)). A fluorescence-labeled preS1 peptide (preS1 probe) to evaluate the attachment of preS1 to host cells was used to observe whether treatment with WD1, WL2, and WL4 reduced the attachment of labeled preS1 to cells in a dose-dependent manner. This was comparable with the effect of CsA used as a positive control (Figure 10(c): quantified data and Figure 10(d): picture). Taken together, the results from the Hep38.7-Tet assay and the preS1 binding assay strongly support the hypothesis that WD1, WL2, and WL4 inhibit preS1-mediated HBV attachment to host cells via binding to NTCP.

The endogenous function of NTCP involves sodium-dependent bile acid uptake (Anwer, M.S. & Stieger, B. Sodium-dependent bile salt transporters of the SLC10A transporter family: more than solute transporters. Pflugers Archiv : European journal of physiology 466, 77-89 (2014), and Hagenbuch, B., Stieger, B., Foguet, M., Lubbert, H. & Meier, P.J. Functional expression cloning and characterization of the hepatocyte Na+/bile acid cotransport system. Proceedings of the National Academy of Sciences of the United States of America 88, 10629-10633 (1991)). The abovementioned HBV entry inhibitors have been shown to inhibit this activity (Konig, A. et al. Kinetics of the bile acid transporter and hepatitis B virus receptor Na+/taurocholate cotransporting polypeptide (NTCP) in hepatocytes. Journal of hepatology 61, 867-875 (2014)., Ni, Y. et al. Hepatitis B and D viruses exploit sodium taurocholate co-transporting polypeptide for species-specific entry into hepatocytes. Gastroenterology 146, 1070-1083 (2014)., Watashi, K. et al. Cyclosporin A and its analogs inhibit hepatitis B virus entry into cultured hepatocytes through targeting a membrane transporter, sodium taurocholate cotransporting polypeptide (NTCP). Hepatology 59, 1726-1737 (2014)., Yan, H. et al. Viral entry of hepatitis B and D viruses and bile salts transportation share common molecular determinants on sodium taurocholate cotransporting polypeptide. Journal of virology 88, 3273-3284 (2014)., Nkongolo, S. et al. Cyclosporin A inhibits hepatitis B and hepatitis D virus entry by cyclophilin-independent interference with the NTCP receptor. Journal of hepatology 60, 723-731 (2014)., Shimura, S. et al. Cyclosporin derivatives inhibit hepatitis B virus entry without interfering with NTCP transporter activity. Journal of hepatology 66, 685-692 (2017), and Kaneko, M. et al. A Novel Tricyclic Polyketide, Vanitaracin A, Specifically Inhibits the Entry of Hepatitis B and D Viruses by Targeting Sodium Taurocholate Cotransporting Polypeptide. Journal of virology 89, 11945-11953 (2015)). However, when NTCP transporter activity in HepG2-hNTCP-C4 cells was evaluated using [³H]-taurocholic acid (TCA) as a substrate in the presence or absence of WD1, WL2, and WL4, no significant inhibition of NTCP transporter activity was observed, even at a peptide concentration of 30 µM (which was approximately solubility in aqueous media) (Figure 4(a)). CsA, by contrast, strongly inhibited NTCP-mediated TCA uptake even at a tenth of this concentration, despite exhibiting rather less anti-HBV activity than that of the 3 cyclic peptides. Thus, WD1, WL2, and WL4 specifically inhibited HBV entry without affecting bile acid uptake, demonstrating that the viral receptor function of NTCP can serve as a target of drug development independently of its bile acid transporter function.

### Inhibition of viral entry is dependent on NTCP and widely acts on various genotypes

Next, the effect of WD1, WL2, and WL4 on various HBV strains and the related HDV was evaluated. As shown in Figures 4(b) and 4(c), the 3 peptides significantly inhibited infection by HDV (which enters cells in a NTCP-dependent manner, as in HBV) but does not affect infection by HCV (which enters cells in an NTCP-independent manner) (Yan, H. et al. Sodium taurocholate cotransporting polypeptide is a functional receptor for human hepatitis B and D virus. eLife 1, e00049 (2012)). In conclusion, these peptides were found to inhibit NTCP-dependent viral infection. In order to evaluate the clinical applicability of these peptides, the anti-HBV effect of WD1 and WL4, the two most potent peptides, was examined on various different HBV isolates. In addition to the previously observed antiviral effect on HBV genotype D (Figure 1), WD1 and WL4 were effective against all the HBV genotypes tested (genotypes A, B, and C) (Figure 4(d)). Moreover, these peptides also significantly inhibited infection by a HBV isolate carrying L180M/S202G/M204V mutations, which is resistant to nucleoside analogs, and a HBV isolate having G145R substitution, which allows vaccine escape. Thus, it was concluded that the thioether-cyclic peptides identified in the present study are broadly active against various HBV strains of clinical concern.

## Claims

1. A sodium taurocholate cotransporting polypeptide (NTCP) inhibitor comprising a cyclic peptide having a Xw1-W-Xw2-W structure, wherein Xw1 and Xw2 are each independently T, S, I, L, or V, or a N-alkylamino acid thereof, or having a P-Xp1-Xp2-H structure, wherein Xp1 is any amino acid, and Xp2 is I, L, or V, or a N-alkylamino acid thereof, or a pharmaceutically acceptable salt thereof.

2. The NTCP inhibitor according to claim 1, wherein the cyclic peptide has a Xw1-W-Xw2-W-Xw3-W structure, wherein Xw1 and Xw2 are as defined above, and Xw3 is T, S, I, L, or V, or a N-alkylamino acid thereof.

3. The NTCP inhibitor according to claim 1 or 2, wherein Xw1 is T or L or a N-alkylamino acid thereof, and Xw2 is T or I or a N-alkylamino acid thereof.

4. The NTCP inhibitor according to claim 1, wherein the cyclic peptide has a P-Xp1-Xp2-H-Xp3-F structure, wherein Xp1 and Xp2 are as defined above, and Xp3 is I, L, or V, or a N-alkylamino acid thereof.

5. The NTCP inhibitor according to claim 4, wherein Xp2 and Xp3 are each independently I or L or a N-alkylamino acid thereof.

6. The NTCP inhibitor according to any one of claims 1 to 5, wherein the cyclic peptide has a N-CO-CH₂-S structure.

7. The NTCP inhibitor according to claim 6, wherein the N is derived from an amino group of tryptophan.

8. The NTCP inhibitor according to claim 6 or 7, wherein the S is derived from a thiol group of cysteine.

9. The NTCP inhibitor according to any one of claims 1 to 8, wherein the cyclic peptide has a cyclic structure having from 10 to 20 amino acids.

10. The NTCP inhibitor according to any one of claims 1 to 9, wherein the NTCP is human NTCP.

11. The NTCP inhibitor according to any one of claims 1 to 10, wherein the NTCP inhibitor is an inhibitor of entry of hepatitis B virus (HBV) or hepatitis D virus (HDV) into cells.

12. A pharmaceutical composition comprising a NTCP inhibitor according to any one of claims 1 to 11.

13. The pharmaceutical composition according to claim 12 for use in treatment or prevention of a disease related to hepatitis B virus or hepatitis D virus.


SEQUENCE LISTING

<110>  The University of Tokyo
       Japan as Represented by Director-General of National
       Institute of Infectious Disease

<120>  NTCP Inhibitor

<130>  007713696

<140>  EP 19738828.3
<141>  2019-01-11

<150>  PCT/JP2019/000699
<151>  2019-01-11

<150>  JP 2018-002814
<151>  2018-01-11

<160>  73

<170>  PatentIn version 3.5

<210>  1
<211>  4
<212>  PRT
<213>  Artificial Sequence

<220>
<223>  partial structure of cyclic peptide


<220>
<221>  misc_feature
<222>  (1)..(1)
<223>  Xaa is Thr, Ser, Ile, Leu or Val, or N-Alkyl amino acid thereof

<220>
<221>  misc_feature
<222>  (3)..(3)
<223>  Xaa is Thr, Ser, Ile, Leu or Val, or N-Alkyl amino acid thereof

<400>  1

Xaa Trp Xaa Trp
1


<210>  2
<211>  4
<212>  PRT
<213>  Artificial Sequence

<220>
<223>  partial structure of cyclic peptide


<220>
<221>  misc_feature
<222>  (2)..(2)
<223>  Xaa can be any amino acid, or N-Alkyl amino acid thereof

<220>

<221>  misc_feature
<222>  (3)..(3)
<223>  Xaa is Ile, Leu or Val, or N-Alkyl amino acid thereof

<400>  2

Pro Xaa Xaa His
1


<210>  3
<211>  6
<212>  PRT
<213>  Artificial Sequence

<220>
<223>  partial structure of cyclic peptide


<220>
<221>  misc_feature
<222>  (1)..(1)
<223>  Xaa is Thr, Ser, Ile, Leu or Val, or N-Alkyl amino acid thereof

<220>
<221>  misc_feature
<222>  (3)..(3)
<223>  Xaa is Thr, Ser, Ile, Leu or Val, or N-Alkyl amino acid thereof

<220>
<221>  misc_feature
<222>  (5)..(5)
<223>  Xaa is Thr, Ser, Ile, Leu or Val, or N-Alkyl amino acid thereof

<400>  3

Xaa Trp Xaa Trp Xaa Trp
1               5


<210>  4
<211>  6
<212>  PRT
<213>  Artificial Sequence

<220>
<223>  partial structure of cyclic peptide


<220>
<221>  misc_feature
<222>  (2)..(2)
<223>  Xaa can be any amino acid

<220>
<221>  misc_feature
<222>  (3)..(3)
<223>  Xaa is Ile, Leu or Val

<220>
<221>  misc_feature
<222>  (5)..(5)
<223>  Xaa is Ile, Leu or Val

<400>  4

Pro Xaa Xaa His Xaa Phe
1               5



<210>  5
<211>  14
<212>  PRT
<213>  Artificial Sequence

<220>
<223>  variable region of cyclic peptide

<400>  5

Ala Leu Trp Ile Trp Pro Gln Val Tyr Trp Thr Arg Phe Ser
1               5                   10



<210>  6
<211>  14
<212>  PRT
<213>  Artificial Sequence

<220>
<223>  variable region of cyclic peptide

<400>  6

Trp Trp Ala Ile Asn Pro His Ile His Val Phe Thr Trp Pro
1               5                   10



<210>  7
<211>  14
<212>  PRT
<213>  Artificial Sequence

<220>
<223>  variable region of cyclic peptide

<400>  7

Ile Leu Val Tyr Pro Glu Leu His Leu Phe Leu Leu Tyr Ser
1               5                   10



<210>  8
<211>  15
<212>  PRT
<213>  Artificial Sequence

<220>
<223>  variable region of cyclic peptide

<400>  8

Leu Tyr Thr Trp Thr Trp His Ser Asn Trp Thr Val Thr Ser Leu
1               5                   10                  15

<210>  9
<211>  15
<212>  PRT
<213>  Artificial Sequence

<220>
<223>  variable region of cyclic peptide

<400>  9

Leu Tyr Leu Trp Thr Trp Pro Asn Thr Ser Tyr Ala Tyr Asn Val
1               5                   10                  15


<210>  10
<211>  12
<212>  PRT
<213>  Artificial Sequence

<220>
<223>  variable region of cyclic peptide

<400>  10

Ser Tyr Ile Asp Thr Phe Thr Trp Ile Trp Leu Tyr
1               5                   10


<210>  11
<211>  12
<212>  PRT
<213>  Artificial Sequence

<220>
<223>  variable region of cyclic peptide


<220>
<221>  MOD_RES
<222>  (5)..(5)
<223>  N-MethylGlycine

<400>  11

Phe Ile Ile Val Gly Thr Trp Thr Trp Thr Trp Ser
1               5                   10


<210>  12
<211>  13
<212>  PRT
<213>  Artificial Sequence

<220>
<223>  variable region of cyclic peptide


<220>
<221>  MOD_RES
<222>  (5)..(6)
<223>  N-MethylGlycine

<400>  12

Thr Trp Ile Trp Gly Gly Leu Tyr Val Val Tyr Phe Pro
1               5                   10

<210>  13
<211>  14
<212>  PRT
<213>  Artificial Sequence

<220>
<223>  variable region of cyclic peptide

<220>
<221>  MOD_RES
<222>  (7)..(7)
<223>  N-MethylSerine

<220>
<221>  MOD_RES
<222>  (13)..(13)
<223>  N-Methyl-4-O-MethylTyrosine

<220>
<221>  MOD_RES
<222>  (14)..(14)
<223>  N-MethylSerine

<400>  13

Ala Leu Ile Trp Thr Trp Ser Gln Tyr Tyr Gln Tyr Tyr Ser
1               5                   10

<210>  14
<211>  13
<212>  PRT
<213>  Artificial Sequence

<220>
<223>  variable region of cyclic peptide

<220>
<221>  MOD_RES
<222>  (13)..(13)
<223>  N-MethylGlycine

<400>  14

Thr Phe Thr Tyr Ala Gln Ile Trp Ile Trp Tyr Tyr Gly
1               5                   10

<210>  15
<211>  12
<212>  PRT
<213>  Artificial Sequence

<220>
<223>  variable region of cyclic peptide

<220>
<221>  MOD_RES
<222>  (6)..(6)
<223>  N-MethylGlycine

<400>  15

Thr Tyr Lys Tyr Ser Gly Ile Trp Ile Trp Ile Trp
1               5                   10


<210>  16
<211>  10
<212>  PRT
<213>  Artificial Sequence

<220>
<223>  variable region of cyclic peptide

<400>  16

Ile Trp Ile Trp Pro Gly Tyr Val Ile Tyr
1               5                   10


<210>  17
<211>  12
<212>  PRT
<213>  Artificial Sequence

<220>
<223>  variable region of cyclic peptide


<220>
<221>  MOD_RES
<222>  (6)..(6)
<223>  N-MethylSerine

<400>  17

Thr Trp Thr Trp His Ser Ala Tyr Leu Tyr Val Phe
1               5                   10


<210>  18
<211>  11
<212>  PRT
<213>  Artificial Sequence

<220>
<223>  variable region of cyclic peptide

<400>  18

Val Ile Trp Ile Trp Pro Asn His Phe Tyr Tyr
1               5                   10


<210>  19
<211>  12
<212>  PRT

<213>  Artificial Sequence

<220>
<223>  variable region of cyclic peptide

<400>  19

Thr Trp Thr Trp Gln Asn Ser Phe Ile Trp Ile Tyr
1               5                   10


<210>  20
<211>  12
<212>  PRT
<213>  Artificial Sequence

<220>
<223>  variable region of cyclic peptide


<220>
<221>  MOD_RES
<222>  (6)..(6)
<223>  N-MethylSerine

<400>  20

Thr Trp Thr Trp Asn Ser Ala Phe Leu Tyr Val Tyr
1               5                   10


<210>  21
<211>  13
<212>  PRT
<213>  Artificial Sequence

<220>
<223>  variable region of cyclic peptide


<220>
<221>  MOD_RES
<222>  (7)..(7)
<223>  N-MethylSerine

<400>  21

Asn Tyr Ile Tyr Tyr Pro Ser Ile His Ile Phe Tyr Leu
1               5                   10


<210>  22
<211>  13
<212>  PRT
<213>  Artificial Sequence

<220>
<223>  variable region of cyclic peptide


<220>
<221>  MOD_RES
<222>  (6)..(6)

<223>  N-MethylSerine

<220>
<221>  MOD_RES
<222>  (13)..(13)
<223>  N-MethylAlanine

<400>  22

His Trp Phe Tyr Pro Ser Ile His Val Phe Thr Trp Ala
1               5                   10


<210>  23
<211>  22
<212>  DNA
<213>  Artificial Sequence

<220>
<223>  Primer T7ex5.F22

<400>  23
ggcgtaatac gactcactat ag                                                22


<210>  24
<211>  36
<212>  DNA
<213>  Artificial Sequence

<220>
<223>  Primer Fx5'.F36

<400>  24
gtaatacgac tcactatagg atcgaaagat ttccgc                                 36


<210>  25
<211>  46
<212>  DNA
<213>  Artificial Sequence

<220>
<223>  Primer dFx.R46

<400>  25
acctaacgcc atgtaccctt tcggggatgc ggaaatcttt cgatcc                      46


<210>  26
<211>  19
<212>  DNA
<213>  Artificial Sequence

<220>
<223>  Primer dFx.R19

<400>  26
acctaacgcc atgtaccct                                                    19


<210>  27
<211>  45

<212>  DNA
<213>  Artificial Sequence

<220>
<223>  Primer eFx.R45

<400>  27
acctaacgct aatccccttt cggggccgcg gaaatctttc gatcc                    45


<210>  28
<211>  18
<212>  DNA
<213>  Artificial Sequence

<220>
<223>  Primer eFx.R18

<400>  28
acctaacgct aatcccct                                                 18


<210>  29
<211>  20
<212>  DNA
<213>  Artificial Sequence

<220>
<223>  Primer EnAsn-3'.R20

<400>  29
tggcggctct gactggactc                                               20


<210>  30
<211>  38
<212>  DNA
<213>  Artificial Sequence

<220>
<223>  Primer EnAsn-3'.R38

<400>  30
tggcggctct gactggactc gaaccagtga catacgga                           38


<210>  31
<211>  49
<212>  DNA
<213>  Artificial Sequence

<220>
<223>  Primer EnAsn-5'.F49

<400>  31
gtaatacgac tcactatagg ctctgtagtt cagtcggtag aacggcgga               49


<210>  32
<211>  43
<212>  DNA
<213>  Artificial Sequence

<220>
<223>  Primer EnAsn NNN.R43

<220>
<221>  misc_feature
<222>  (21)..(23)
<223>  n is a, c, g, or t

<400>  32
gaaccagtga catacggatt nnnagtccgc cgttctaccg act                         43

<210>  33
<211>  66
<212>  DNA
<213>  Artificial Sequence

<220>
<223>  Primer NNKpool-R1n6.R66

<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<400>  33
ctagctgccg ctgccgctgc cgcannmnnm nnmnnmnnmn nmcatatgta tatctccttc       60

ttaaag                                                                  66

<210>  34
<211>  69
<212>  DNA
<213>  Artificial Sequence

<220>
<223>  Primer NNKpool-R1n7.R69


<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<400>  34
ctagctgccg ctgccgctgc cgcannmnnm nnmnnmnnmn nmnnmcatat gtatatctcc      60

ttcttaaag                                                              69


<210>  35
<211>  72
<212>  DNA
<213>  Artificial Sequence

<220>
<223>  Primer NNKpool-R1n8.R72


<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<220>
<221>  misc_feature
<222>  (46)..(47)
<223>  n is a, c, g, or t

<400>  35
ctagctgccg ctgccgctgc cgcannmnnm nnmnnmnnmn nmnnmnnmca tatgtatatc      60

tccttcttaa ag                                                          72


<210>  36
<211>  75
<212>  DNA
<213>  Artificial Sequence

<220>
<223>  Primer NNKpool-R1n9.R75



<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature

<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<220>
<221>  misc_feature
<222>  (46)..(47)
<223>  n is a, c, g, or t

<220>
<221>  misc_feature
<222>  (49)..(50)
<223>  n is a, c, g, or t

<400>  36
ctagctgccg ctgccgctgc cgcannmnnm nnmnnmnnmn nmnnmnnmnn mcatatgtat      60

atctccttct taaag                                                        75


<210>  37
<211>  78
<212>  DNA
<213>  Artificial Sequence

<220>
<223>  Primer NNKpool-R1n10.R78



<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<220>
<221>  misc_feature
<222>  (46)..(47)
<223>  n is a, c, g, or t

<220>
<221>  misc_feature
<222>  (49)..(50)
<223>  n is a, c, g, or t

<220>
<221>  misc_feature
<222>  (52)..(53)
<223>  n is a, c, g, or t

<400>  37
ctagctgccg ctgccgctgc cgcannmnnm nnmnnmnnmn nmnnmnnmnn mnnmcatatg        60

tatatctcct tcttaaag                                                      78


<210>  38
<211>  81
<212>  DNA
<213>  Artificial Sequence

<220>
<223>  Primer NNKpool-R1n11.R81


<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)

<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<220>
<221>  misc_feature
<222>  (46)..(47)
<223>  n is a, c, g, or t

<220>
<221>  misc_feature
<222>  (49)..(50)
<223>  n is a, c, g, or t

<220>
<221>  misc_feature
<222>  (52)..(53)
<223>  n is a, c, g, or t

<220>
<221>  misc_feature
<222>  (55)..(56)
<223>  n is a, c, g, or t

<400>  38
ctagctgccg ctgccgctgc cgcannmnnm nnmnnmnnmn nmnnmnnmnn mnnmnnmcat      60

atgtatatct ccttcttaaa g                                                81


<210>  39
<211>  84
<212>  DNA
<213>  Artificial Sequence

<220>
<223>  Primer NNKpool-R1n12.R84


<220>
<221>  misc_feature
<222>  (25)..(26)
<223>  n is a, c, g, or t

<220>
<221>  misc_feature
<222>  (28)..(29)
<223>  n is a, c, g, or t

<220>

<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<220>
<221>  misc_feature
<222>  (46)..(47)
<223>  n is a, c, g, or t

<220>
<221>  misc_feature
<222>  (49)..(50)
<223>  n is a, c, g, or t

<220>
<221>  misc_feature
<222>  (52)..(53)
<223>  n is a, c, g, or t

<220>
<221>  misc_feature
<222>  (55)..(56)
<223>  n is a, c, g, or t

<220>
<221>  misc_feature
<222>  (58)..(59)
<223>  n is a, c, g, or t

<400>  39
ctagctgccg ctgccgctgc cgcannmnnm nnmnnmnnmn nmnnmnnmnn mnnmnnmnnm      60

catatgtata tctccttctt aaag                                              84


<210>  40
<211>  87
<212>  DNA
<213>  Artificial Sequence

<220>
<223>  Primer NNKpool-R1n13.R87

<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<220>
<221>  misc_feature
<222>  (46)..(47)
<223>  n is a, c, g, or t

<220>
<221>  misc_feature
<222>  (49)..(50)
<223>  n is a, c, g, or t

<220>
<221>  misc_feature
<222>  (52)..(53)
<223>  n is a, c, g, or t

<220>
<221>  misc_feature
<222>  (55)..(56)
<223>  n is a, c, g, or t

<220>
<221>  misc_feature
<222>  (58)..(59)
<223>  n is a, c, g, or t

<220>
<221>  misc_feature

<222>  (61)..(62)
<223>  n is a, c, g, or t

<400>  40
ctagctgccg ctgccgctgc cgcannmnnm nnmnnmnnmn nmnnmnnmnn mnnmnnmnnm      60

nnmcatatgt atatctcctt cttaaag                                          87


<210>  41
<211>  90
<212>  DNA
<213>  Artificial Sequence

<220>
<223>  Primer NNKpool-R1n14.R90


<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<220>
<221>  misc_feature
<222>  (46)..(47)
<223>  n is a, c, g, or t

<220>
<221>  misc_feature
<222>  (49)..(50)
<223>  n is a, c, g, or t

<220>
<221>  misc_feature
<222>  (52)..(53)
<223>  n is a, c, g, or t

<220>
<221>  misc_feature
<222>  (55)..(56)
<223>  n is a, c, g, or t

<220>
<221>  misc_feature
<222>  (58)..(59)
<223>  n is a, c, g, or t

<220>
<221>  misc_feature
<222>  (61)..(62)
<223>  n is a, c, g, or t

<220>
<221>  misc_feature
<222>  (64)..(65)
<223>  n is a, c, g, or t

<400>  41
ctagctgccg ctgccgctgc cgcannmnnm nnmnnmnnmn nmnnmnnmnn mnnmnnmnnm        60

nnmnnmcata tgtatatctc cttcttaaag                                          90


<210>  42
<211>  93
<212>  DNA
<213>  Artificial Sequence

<220>
<223>  Primer NNKpool-R1n15.R93



<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)

<223>  n is a, c, g, or t

<220>
<221>  misc_feature
<222>  (40)..(41)
<223>  n is a, c, g, or t

<220>
<221>  misc_feature
<222>  (43)..(44)
<223>  n is a, c, g, or t

<220>
<221>  misc_feature
<222>  (46)..(47)
<223>  n is a, c, g, or t

<220>
<221>  misc_feature
<222>  (49)..(50)
<223>  n is a, c, g, or t

<220>
<221>  misc_feature
<222>  (52)..(53)
<223>  n is a, c, g, or t

<220>
<221>  misc_feature
<222>  (55)..(56)
<223>  n is a, c, g, or t

<220>
<221>  misc_feature
<222>  (58)..(59)
<223>  n is a, c, g, or t

<220>
<221>  misc_feature
<222>  (61)..(62)
<223>  n is a, c, g, or t

<220>
<221>  misc_feature
<222>  (64)..(65)
<223>  n is a, c, g, or t

<220>
<221>  misc_feature
<222>  (67)..(68)
<223>  n is a, c, g, or t

<400>  42
ctagctgccg ctgccgctgc cgcannmnnm nnmnnmnnmn nmnnmnnmnn mnnmnnmnnm        60

nnmnnmnnmc atatgtatat ctccttctta aag                                     93


<210>  43
<211>  39
<212>  DNA
<213>  Artificial Sequence

<220>
<223>  Primer CGS3an13.R39

<400>  43
tttccgcccc ccgtcctagc tgccgctgcc gctgccgca                              39


<210>  44
<211>  16
<212>  DNA
<213>  Artificial Sequence

<220>
<223>  Primer Puromycin linker


<220>
<221>  modified_base
<222>  (16)..(16)
<223>  cytosine-(PEG18)5-cytosine-cytosine-purine

<400>  44
ctcccgcccc ccgtcc                                                       16


<210>  45
<211>  46
<212>  DNA
<213>  Artificial Sequence

<220>
<223>  Primer T7g10M.F46

<400>  45
taatacgact cactataggg ttaactttaa gaaggagata tacata                      46


<210>  46
<211>  39
<212>  DNA
<213>  Artificial Sequence

<220>
<223>  Primer CTQ3an13.R39

<400>  46
tttccgcccc ccgtcctact gtgtctgtgt ctgtgtgca                              39


<210>  47
<211>  45
<212>  DNA
<213>  Artificial Sequence

<220>
<223>  Primer PXLHLF-NNS-2.F45


<220>
<221>  misc_feature
<222>  (25)..(26)
<223>  n is a, c, g, or t

<220>
<221>  misc_feature
<222>  (31)..(32)
<223>  n is a, c, g, or t

<400>  47
ctttaagaag gagatataca tatgnnsccg nnsvtscacv tsttc                    45


<210>  48
<211>  48
<212>  DNA
<213>  Artificial Sequence

<220>
<223>  Primer PXLHLF-NNS-2.F48


<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (34)..(35)
<223>  n is a, c, g, or t

<400>  48
ctttaagaag gagatataca tatgnnsnns ccgnnsvtsc acvtsttc                 48


<210>  49
<211>  51
<212>  DNA
<213>  Artificial Sequence

<220>
<223>  Primer PXLHLF-NNS-2.F51


<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature

<222>  (37)..(38)
<223>  n is a, c, g, or t

<400>  49
ctttaagaag gagatataca tatgnnsnns nnsccgnnsv tscacvtstt c                51


<210>  50
<211>  54
<212>  DNA
<213>  Artificial Sequence

<220>
<223>  Primer PXLHLF-NNS-2.F54


<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (40)..(41)
<223>  n is a, c, g, or t

<400>  50
ctttaagaag gagatataca tatgnnsnns nnsnnsccgn nsvtscacvt sttc             54


<210>  51
<211>  57
<212>  DNA
<213>  Artificial Sequence

<220>
<223>  Primer PXLHLF-NNS-2.F57


<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (43)..(44)
<223>  n is a, c, g, or t

<400>  51
ctttaagaag gagatataca tatgnnsnns nnsnnsnnsc cgnnsvtsca cvtsttc          57


<210>  52
<211>  45
<212>  DNA
<213>  Artificial Sequence

<220>
<223>  Primer PXLHLF-NNS-2.R45


<220>
<221>  misc_feature
<222>  (26)..(27)
<223>  n is a, c, g, or t

<220>
<221>  misc_feature
<222>  (41)..(42)
<223>  n is a, c, g, or t

<400>  52
ctactgtgtc tgtgtctgtg tgcasnngaa sabgtgsabs nncgg                       45


<210>  53
<211>  48
<212>  DNA
<213>  Artificial Sequence

<220>
<223>  Primer PXLHLF-NNS-2.R48


<220>
<221>  misc_feature
<222>  (26)..(27)
<223>  n is a, c, g, or t

<220>

<221>  misc_feature
<222>  (29)..(30)
<223>  n is a, c, g, or t

<220>
<221>  misc_feature
<222>  (44)..(45)
<223>  n is a, c, g, or t

<400>  53
ctactgtgtc tgtgtctgtg tgcasnnsnn gaasabgtgs absnncgg                48


<210>  54
<211>  51
<212>  DNA
<213>  Artificial Sequence

<220>
<223>  Primer PXLHLF-NNS-2.R51


<220>
<221>  misc_feature
<222>  (26)..(27)
<223>  n is a, c, g, or t

<220>
<221>  misc_feature
<222>  (29)..(30)
<223>  n is a, c, g, or t

<220>
<221>  misc_feature
<222>  (32)..(33)
<223>  n is a, c, g, or t

<220>
<221>  misc_feature
<222>  (47)..(48)
<223>  n is a, c, g, or t

<400>  54
ctactgtgtc tgtgtctgtg tgcasnnsnn snngaasabg tgsabsnncg g            51


<210>  55
<211>  54
<212>  DNA
<213>  Artificial Sequence

<220>
<223>  Primer PXLHLF-NNS-2.R54


<220>
<221>  misc_feature
<222>  (26)..(27)
<223>  n is a, c, g, or t

<220>
<221>  misc_feature
<222>  (29)..(30)

<223>  n is a, c, g, or t

<220>
<221>  misc_feature
<222>  (32)..(33)
<223>  n is a, c, g, or t

<220>
<221>  misc_feature
<222>  (35)..(36)
<223>  n is a, c, g, or t

<220>
<221>  misc_feature
<222>  (50)..(51)
<223>  n is a, c, g, or t

<400>  55
ctactgtgtc tgtgtctgtg tgcasnnsnn snnsnngaas abgtgsabsn ncgg          54


<210>  56
<211>  36
<212>  DNA
<213>  Artificial Sequence

<220>
<223>  Primer TWTW-NNS.F36

<400>  56
ctttaagaag gagatataca tatgaystgg aystgg                              36


<210>  57
<211>  39
<212>  DNA
<213>  Artificial Sequence

<220>
<223>  Primer TWTW-NNS.F39


<220>
<221>  misc_feature
<222>  (25)..(26)
<223>  n is a, c, g, or t

<400>  57
ctttaagaag gagatataca tatgnnsays tggaystgg                           39


<210>  58
<211>  42
<212>  DNA
<213>  Artificial Sequence

<220>
<223>  Primer TWTW-NNS.F42


<220>
<221>  misc_feature
<222>  (25)..(26)

<223>  n is a, c, g, or t

<220>
<221>  misc_feature
<222>  (28)..(29)
<223>  n is a, c, g, or t

<400>  58
ctttaagaag gagatataca tatgnnsnns aystggayst gg                          42


<210>  59
<211>  45
<212>  DNA
<213>  Artificial Sequence

<220>
<223>  Primer TWTW-NNS.F45


<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<400>  59
ctttaagaag gagatataca tatgnnsnns nnsaystgga ystgg                       45


<210>  60
<211>  48
<212>  DNA
<213>  Artificial Sequence

<220>
<223>  Primer TWTW-NNS.F48


<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)
<223>  n is a, c, g, or t

<400>  60
ctttaagaag gagatataca tatgnnsnns nnsnnsayst ggaystgg                    48


<210>  61
<211>  51
<212>  DNA
<213>  Artificial Sequence

<220>
<223>  Primer TWTW-NNS.F51



<220>
<221>  misc_feature
<222>  (25)..(26)
<223>  n is a, c, g, or t

<220>
<221>  misc_feature
<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<400>  61
ctttaagaag gagatataca tatgnnsnns nnsnnsnnsa ystggaystg g                51


<210>  62
<211>  54
<212>  DNA
<213>  Artificial Sequence

<220>
<223>  Primer TWTW-NNS.F54



<220>
<221>  misc_feature
<222>  (25)..(26)
<223>  n is a, c, g, or t

<220>
<221>  misc_feature

<222>  (28)..(29)
<223>  n is a, c, g, or t

<220>
<221>  misc_feature
<222>  (31)..(32)
<223>  n is a, c, g, or t

<220>
<221>  misc_feature
<222>  (34)..(35)
<223>  n is a, c, g, or t

<220>
<221>  misc_feature
<222>  (37)..(38)
<223>  n is a, c, g, or t

<220>
<221>  misc_feature
<222>  (40)..(41)
<223>  n is a, c, g, or t

<400>  62
ctttaagaag gagatataca tatgnnsnns nnsnnsnnsn nsaystggay stgg          54


<210>  63
<211>  36
<212>  DNA
<213>  Artificial Sequence

<220>
<223>  Primer TWTW-NNS.R36

<400>  63
ctactgtgtc tgtgtctgtg tgcaccasrt ccasrt                              36


<210>  64
<211>  39
<212>  DNA
<213>  Artificial Sequence

<220>
<223>  Primer TWTW-NNS.R39


<220>
<221>  misc_feature
<222>  (26)..(27)
<223>  n is a, c, g, or t

<400>  64
ctactgtgtc tgtgtctgtg tgcasnncca srtccasrt                           39


<210>  65
<211>  42
<212>  DNA
<213>  Artificial Sequence

<220>

<223>  Primer TWTW-NNS.R42

<220>
<221>  misc_feature
<222>  (26)..(27)
<223>  n is a, c, g, or t

<220>
<221>  misc_feature
<222>  (29)..(30)
<223>  n is a, c, g, or t

<400>  65
ctactgtgtc tgtgtctgtg tgcasnnsnn ccasrtccas rt                    42


<210>  66
<211>  45
<212>  DNA
<213>  Artificial Sequence

<220>
<223>  Primer TWTW-NNS.R45


<220>
<221>  misc_feature
<222>  (26)..(27)
<223>  n is a, c, g, or t

<220>
<221>  misc_feature
<222>  (29)..(30)
<223>  n is a, c, g, or t

<220>
<221>  misc_feature
<222>  (32)..(33)
<223>  n is a, c, g, or t

<400>  66
ctactgtgtc tgtgtctgtg tgcasnnsnn snnccasrtc casrt                 45


<210>  67
<211>  48
<212>  DNA
<213>  Artificial Sequence

<220>
<223>  Primer TWTW-NNS.R48


<220>
<221>  misc_feature
<222>  (26)..(27)
<223>  n is a, c, g, or t

<220>
<221>  misc_feature
<222>  (29)..(30)
<223>  n is a, c, g, or t

<220>
<221>  misc_feature
<222>  (32)..(33)
<223>  n is a, c, g, or t

<220>
<221>  misc_feature
<222>  (35)..(36)
<223>  n is a, c, g, or t

<400>  67
ctactgtgtc tgtgtctgtg tgcasnnsnn snnsnnccas rtccasrt                48


<210>  68
<211>  51
<212>  DNA
<213>  Artificial Sequence

<220>
<223>  Primer TWTW-NNS.R51


<220>
<221>  misc_feature
<222>  (26)..(27)
<223>  n is a, c, g, or t

<220>
<221>  misc_feature
<222>  (29)..(30)
<223>  n is a, c, g, or t

<220>
<221>  misc_feature
<222>  (32)..(33)
<223>  n is a, c, g, or t

<220>
<221>  misc_feature
<222>  (35)..(36)
<223>  n is a, c, g, or t

<220>
<221>  misc_feature
<222>  (38)..(39)
<223>  n is a, c, g, or t

<400>  68
ctactgtgtc tgtgtctgtg tgcasnnsnn snnsnnsnnc casrtccasr t            51


<210>  69
<211>  54
<212>  DNA
<213>  Artificial Sequence

<220>
<223>  Primer TWTW-NNS.R54


<220>

<221>  misc_feature
<222>  (26)..(27)
<223>  n is a, c, g, or t

<220>
<221>  misc_feature
<222>  (29)..(30)
<223>  n is a, c, g, or t

<220>
<221>  misc_feature
<222>  (32)..(33)
<223>  n is a, c, g, or t

<220>
<221>  misc_feature
<222>  (35)..(36)
<223>  n is a, c, g, or t

<220>
<221>  misc_feature
<222>  (38)..(39)
<223>  n is a, c, g, or t

<220>
<221>  misc_feature
<222>  (41)..(42)
<223>  n is a, c, g, or t

<400>  69
ctactgtgtc tgtgtctgtg tgcasnnsnn snnsnnsnns nnccasrtcc asrt          54


<210>  70
<211>  57
<212>  DNA
<213>  Artificial Sequence

<220>
<223>  Primer TWTW-NNS.R57



<220>
<221>  misc_feature
<222>  (26)..(27)
<223>  n is a, c, g, or t

<220>
<221>  misc_feature
<222>  (29)..(30)
<223>  n is a, c, g, or t

<220>
<221>  misc_feature
<222>  (32)..(33)
<223>  n is a, c, g, or t

<220>
<221>  misc_feature
<222>  (35)..(36)
<223>  n is a, c, g, or t

<220>

<221>  misc_feature
<222>  (38)..(39)
<223>  n is a, c, g, or t

<220>
<221>  misc_feature
<222>  (41)..(42)
<223>  n is a, c, g, or t

<220>
<221>  misc_feature
<222>  (44)..(45)
<223>  n is a, c, g, or t

<400>  70
ctactgtgtc tgtgtctgtg tgcasnnsnn snnsnnsnns nnsnnccasr tccasrt          57


<210>  71
<211>  60
<212>  DNA
<213>  Artificial Sequence

<220>
<223>  Primer TWTW-NNS.R60


<220>
<221>  misc_feature
<222>  (26)..(27)
<223>  n is a, c, g, or t

<220>
<221>  misc_feature
<222>  (29)..(30)
<223>  n is a, c, g, or t

<220>
<221>  misc_feature
<222>  (32)..(33)
<223>  n is a, c, g, or t

<220>
<221>  misc_feature
<222>  (35)..(36)
<223>  n is a, c, g, or t

<220>
<221>  misc_feature
<222>  (38)..(39)
<223>  n is a, c, g, or t

<220>
<221>  misc_feature
<222>  (41)..(42)
<223>  n is a, c, g, or t

<220>
<221>  misc_feature
<222>  (44)..(45)
<223>  n is a, c, g, or t

<220>

<221>  misc_feature
<222>  (47)..(48)
<223>  n is a, c, g, or t

<400>  71
ctactgtgtc tgtgtctgtg tgcasnnsnn snnsnnsnns nnsnnsnncc asrtccasrt      60


<210>  72
<211>  63
<212>  DNA
<213>  Artificial Sequence

<220>
<223>  Primer TWTW-NNS.R63



<220>
<221>  misc_feature
<222>  (26)..(27)
<223>  n is a, c, g, or t

<220>
<221>  misc_feature
<222>  (29)..(30)
<223>  n is a, c, g, or t

<220>
<221>  misc_feature
<222>  (32)..(33)
<223>  n is a, c, g, or t

<220>
<221>  misc_feature
<222>  (35)..(36)
<223>  n is a, c, g, or t

<220>
<221>  misc_feature
<222>  (38)..(39)
<223>  n is a, c, g, or t

<220>
<221>  misc_feature
<222>  (41)..(42)
<223>  n is a, c, g, or t

<220>
<221>  misc_feature
<222>  (44)..(45)
<223>  n is a, c, g, or t

<220>
<221>  misc_feature
<222>  (47)..(48)
<223>  n is a, c, g, or t

<220>
<221>  misc_feature
<222>  (50)..(51)
<223>  n is a, c, g, or t

<400>  72

ctactgtgtc tgtgtctgtg tgcasnnsnn snnsnnsnns nnsnnsnnsn nccasrtcca 60

srt 63

<210> 73
<211> 349
<212> PRT
<213> Artificial Sequence

<220>
<223> NTCP

<400> 73

Met Glu Ala His Asn Ala Ser Ala Pro Phe Asn Phe Thr Leu Pro Pro
1               5                   10                  15

Asn Phe Gly Lys Arg Pro Thr Asp Leu Ala Leu Ser Val Ile Leu Val
            20                  25                  30

Phe Met Leu Phe Phe Ile Met Leu Ser Leu Gly Cys Thr Met Glu Phe
        35                  40                  45

Ser Lys Ile Lys Ala His Leu Trp Lys Pro Lys Gly Leu Ala Ile Ala
    50                  55                  60

Leu Val Ala Gln Tyr Gly Ile Met Pro Leu Thr Ala Phe Val Leu Gly
65                  70                  75                  80

Lys Val Phe Arg Leu Lys Asn Ile Glu Ala Leu Ala Ile Leu Val Cys
                85                  90                  95

Gly Cys Ser Pro Gly Gly Asn Leu Ser Asn Val Phe Ser Leu Ala Met
            100                 105                 110

Lys Gly Asp Met Asn Leu Ser Ile Val Met Thr Thr Cys Ser Thr Phe
        115                 120                 125

Cys Ala Leu Gly Met Met Pro Leu Leu Leu Tyr Ile Tyr Ser Arg Gly
    130                 135                 140

Ile Tyr Asp Gly Asp Leu Lys Asp Lys Val Pro Tyr Lys Gly Ile Val
145                 150                 155                 160

Ile Ser Leu Val Leu Val Leu Ile Pro Cys Thr Ile Gly Ile Val Leu
                165                 170                 175

Lys Ser Lys Arg Pro Gln Tyr Met Arg Tyr Val Ile Lys Gly Gly Met
            180                 185                 190

Ile Ile Ile Leu Leu Cys Ser Val Ala Val Thr Val Leu Ser Ala Ile
        195                 200                 205

Asn Val Gly Lys Ser Ile Met Phe Ala Met Thr Pro Leu Leu Ile Ala
    210                 215                 220

Thr Ser Ser Leu Met Pro Phe Ile Gly Phe Leu Leu Gly Tyr Val Leu
225                 230                 235                 240

Ser Ala Leu Phe Cys Leu Asn Gly Arg Cys Arg Arg Thr Val Ser Met
                245                 250                 255

Glu Thr Gly Cys Gln Asn Val Gln Leu Cys Ser Thr Ile Leu Asn Val
            260                 265                 270

Ala Phe Pro Pro Glu Val Ile Gly Pro Leu Phe Phe Phe Pro Leu Leu
        275                 280                 285

Tyr Met Ile Phe Gln Leu Gly Glu Gly Leu Leu Leu Ile Ala Ile Phe
    290                 295                 300

Trp Cys Tyr Glu Lys Phe Lys Thr Pro Lys Asp Lys Thr Lys Met Ile
305                 310                 315                 320

Tyr Thr Ala Ala Thr Thr Glu Glu Thr Ile Pro Gly Ala Leu Gly Asn
                325                 330                 335

Gly Thr Tyr Lys Gly Glu Asp Cys Ser Pro Cys Thr Ala
            340                 345